# EUROPEAN PATENT APPLICATION

(11) **EP 4 538 375 A1**
(43) Date of publication of application: **16.04.2025**
(21) Application number: 23818802.3
(22) Date of filing: 30.03.2023
(51) Int. Cl.: C12N 15/113, C12N 15/77, C12N 1/21, C12P 13/08, C12P 13/14, C12R 1/15

(54) **EP6 PROMOTER, RELATED BIOMATERIAL, AND USE THEREOF**

(30) Priority: 10.06.2022 CN 202210650799
(71) Applicant: Ningxia Eppen Biotech Co., Ltd, Yinchuan, Ningxia 750199 (CN)
(72) Inventor: WEI, Aiying, Yinchuan, Ningxia 750199 (CN); MENG, Gang, Yinchuan, Ningxia 750199 (CN); JIA, Huiping, Yinchuan, Ningxia 750199 (CN); MI, Jie, Yinchuan, Ningxia 750199 (CN); YANG, Lipeng, Yinchuan, Ningxia 750199 (CN); SU, Houbo, Yinchuan, Ningxia 750199 (CN); TIAN, Bin, Yinchuan, Ningxia 750199 (CN)
(74) Representative: Winter, Brandl - Partnerschaft mbB
(86) International application number: PCT/CN2023/084969
(87) International publication number: WO 2023/236634

(57) **Abstract**

Provided are an EP6 promoter, a related biomaterial, and use thereof. The sequence of the EP6 promoter is set forth in SEQ ID No. 3 in the sequence listing. The EP6 promoter is used as an exogenous strong promoter to be integrated into bacteria with high-yield amino acids to replace an original promoter of key genes for amino acid synthesis, so that the yield of a corresponding amino acid is improved.

## Description

### Technical Field

The present invention relates to an EP6 promoter, a related biomaterial and use thereof in the field of biotechnologies.

### Background Art

At present, the level of amino acid production in the industry has been greatly improved, but how to further increase the yield of amino acids at a higher level requires a comprehensive consideration of carbon sources required for the growth of bacteria producing amino acids, as well as maximized entry of the carbon sources into an amino acid synthesis pathway in the later stage of fermentation. In order to solve this problem, promoters having different intensities need to be applied to different genes of corresponding amino acid metabolism pathways for bacteria producing a certain amino acid, so as to achieve a balance between the amino acid metabolism pathways and the bacterial growth.

### Summary of the Invention

An object of the present invention is to provide an EP6 promoter. The EP6 promoter can be used to produce an amino acid.

A sequence of the EP6 promoter provided by the present invention is set forth in SEQ ID No. 3 in the sequence listing.

The present invention further provides a biomaterial associated with the EP6 promoter. The biomaterial is any of the followings B1) to B7):
B1) an expression cassette containing the EP6;
B2) a recombinant vector containing the EP6 promoter;
B3) a recombinant vector containing the expression cassette of B1);
B4) a recombinant microorganism containing the EP6 promoter;
B5) a recombinant microorganism containing the expression cassette of B1);
B6) a recombinant microorganism containing the recombinant vector of B2); and
B7) a recombinant microorganism containing the recombinant vector of B3).

In the above-mentioned biomaterial, the expression cassette containing the EP6 promoter of B1) refers to a DNA that can drive the expression of a target gene with the EP6 promoter in a host cell, and this DNA may include not only the target gene, but also a terminator that terminates the transcription of the target gene. Further, the expression cassette may also include an enhancer sequence.

The recombinant vector containing the EP6 promoter may be constructed by using the existing expression vector.

In the above-mentioned biomaterial, the vector may be a plasmid, a cosmid, a bacteriophage or a viral vector.

In the above-mentioned biomaterial, the microorganism may be yeast, bacteria, algae, or fungus. The bacteria may be Corynebacterium glutamicum, Brevibacterium lactofermentum, brevibacterium flavum, Corynebacterium pekinense, Brevibacterium ammoniagenes, Corynebacterium crenatum, or Pantoea.

The present invention further provides use of the EP6 promoter as a promoter.

The present invention further provides use of the EP6 promoter in the production of an amino acid.

The present invention further provides use of a biomaterial in the production of an amino acid.

In the above-mentioned use, the amino acid may be lysine, glutamic acid or valine.

The EP6 promoter of the present invention may be used to produce a variety of products, including but not limited to lysine, glutamic acid and valine in the embodiment; and the products may also be glycine, alanine, leucine, isoleucine, methionine, proline, tryptophan, serine, tyrosine, cysteine, phenylalanine, asparagine, glutamine, threonine, aspartic acid, arginine, histidine, shikimic acid, protocatechuic acid, succinic acid, α-ketoglutarate, citric acid, ornithine, citrulline, etc. During the production of various products, the EP6 promoter of the present invention is placed in the upstream of a gene in a synthesis pathway of a target product; and the EP6 promoter drives the synthesis of the gene in the synthesis pathway of the target product to realize the production of the target product.

The present invention further provides a method for producing an amino acid. The method includes: introducing the EP6 promoter into a biological cell capable of synthesizing a target amino acid, so that the EP6 promoter drives the expression of a gene in a synthesis pathway of a target amino acid in the biological cell to obtain a recombinant biological cell; and culturing the recombinant biological cell to obtain the target amino acid.

In the above-mentioned method, the biological cell may be yeast, bacteria, algae, fungus, a plant cell or an animal cell that can synthesize the target amino acid.

The biological cell is any biological cell that can synthesize the target amino acid.

The bacteria may be Corynebacterium glutamicum, Brevibacterium lactofermentum, brevibacterium flavum, Corynebacterium pekinense, Brevibacterium ammoniagenes, Corynebacterium crenatum, or Pantoea.

In one example of the present invention, the target amino acid is lysine; and the bacteria are Corynebacterium glutamicum CGMCC No. 12856.

The bacteria used in the production of lysine by using the promoter of the present invention include, but are not limited to, Corynebacterium glutamicum CGMCC No. 12856. In the present invention, the EP6 promoter of the present invention is placed in the upstream of a gene in a lysine synthesis pathway of these bacteria, so that the EP6 promoter of the present invention can synthesize lysine by driving the expression of the gene in the lysine synthesis pathway of these bacteria.

In one example of the present invention, the target amino acid is glutamic acid; and the bacteria are Corynebacterium glutamicum CGMCC No. 21220.

The bacteria used in the production of glutamic acid by using the promoter of the present invention include, but are not limited to, Corynebacterium glutamicum CGMCC No. 21220. In the present invention, the EP6 promoter of the present invention is placed in the upstream of a gene in a glutamic acid synthesis pathway of these bacteria, so that the EP6 promoter of the present invention can synthesize glutamic acid by driving the expression of the gene in the glutamic acid synthesis pathway of these bacteria.

In one example of the present invention, the target amino acid is valine; and the bacteria are Corynebacterium glutamicum CGMCC No. 21260.

The bacteria used in the production of valine by using the promoter of the present invention include, but are not limited to, Corynebacterium glutamicum CGMCC No. 21260. In the present invention, the EP6 promoter of the present invention is placed in the upstream of a gene in a valine synthesis pathway of these bacteria, so that the EP6 promoter of the present invention can synthesize valine by driving the expression of the gene in the valine synthesis pathway of these bacteria.

In the above-mentioned method, the target amino acid may be lysine, glutamic acid or valine.

The EP6 promoter of the present invention may be used to produce a variety of products, including but not limited to lysine, glutamic acid and valine in the embodiment; and the products may also be glycine, alanine, leucine, isoleucine, methionine, proline, tryptophan, serine, tyrosine, cysteine, phenylalanine, asparagine, glutamine, threonine, aspartic acid, arginine, histidine, shikimic acid, protocatechuic acid, succinic acid, α-ketoglutarate, citric acid, ornithine, citrulline, etc. During the production of various products, the EP6 promoter of the present invention is placed in the upstream of a gene in a synthesis pathway of a target product; and the EP6 promoter drives the synthesis of the gene in the synthesis pathway of the target product to realize the production of the target product.

In one example of the present invention, the target amino acid is lysine; the bacteria are Corynebacterium glutamicum CGMCC No. 12856; and the gene in the lysine synthesis pathway is a lysA gene (positions 149-1486 of SEQ ID No. 4).

The recombinant biological cell is realized by replacing the EP6 promoter with an original promoter of the lysA gene in Corynebacterium glutamicum CGMCC No. 12856.

In one example of the present invention, the target amino acid is glutamic acid; the bacteria are Corynebacterium glutamicum CGMCC No. 21220; and the gene in the glutamic acid synthesis pathway is a BBD29_14295 gene (positions 37-1161 of SEQ ID No. 11).

The recombinant biological cell is realized by replacing the EP6 promoter with an original promoter of the BBD29_14295 gene in Corynebacterium glutamicum CGMCC No. 21220.

In one example of the present invention, the target amino acid is valine; the bacteria are Corynebacterium glutamicum CGMCC No. 21260; and the gene in the valine synthesis pathway is an ilvC gene (positions 180-1196 of SEQ ID No. 18).

The recombinant biological cell is realized by replacing the EP6 promoter with an original promoter of the ilvC gene in Corynebacterium glutamicum CGMCC No. 21260.

The present invention further provides a product for producing an amino acid, an active ingredient of which is the EP6 promoter or the biomaterial.

Instruction for the preservation of biomaterial.

Classification and naming: Corynebacterium glutamicum.

Strain number: YPGLU001.

Title of preservation unit: General Microbiological Center of China Microbiological Culture Collection Management Committee.

Abbreviation of preservation unit: CGMCC.

Address of preservation unit: No. 3, Courtyard 1, Beichen West Road, Chaoyang District, Beijing, Postal Code: 100101.

Preservation date: November 23, 2020.

Preservation Center registration number: CGMCC No. 21220.

Instruction for the preservation of biomaterial.

Classification and naming: Corynebacterium glutamicum.

Strain number: YPFV1.

Title of preservation unit: General Microbiological Center of China Microbiological Culture Collection Management Committee.

Abbreviation of preservation unit: CGMCC.

Address of preservation unit: No. 3, Courtyard 1, Beichen West Road, Chaoyang District, Beijing, Postal Code: 100101.

Preservation date: November 30, 2020.

Preservation Center registration number: CGMCC No. 21260.

Instruction for the preservation of biomaterial.

Classification and naming: Corynebacterium glutamicum.

Strain number: YP097158.

Title of preservation unit: General Microbiological Center of China Microbiological Culture Collection Management Committee.

Abbreviation of preservation unit: CGMCC.

Address of preservation unit: No. 3, Courtyard 1, Beichen West Road, Chaoyang District, Beijing, Postal Code: 100101.

Preservation date: August 16, 2016.

Preservation Center registration number: CGMCC No. 12856.

### Brief Description of the Drawings

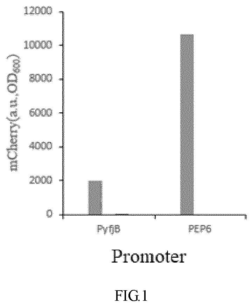

FIG. 1 shows detection results of promoter activities of EP6 and yfjB. PEP6 is an EP6 promoter.

### Detailed Description of the Invention

The present invention is described in further detail below in conjunction with the specific embodiments, and the given examples are only for the purpose of clarifying the present invention, but not for the purpose of limiting the scope of the present invention. The examples provided below may be used as a guide for further improvement by a person of ordinary skill in the art and do not in any way constitute a limitation of the present invention.

The experimental methods in the following examples, unless otherwise specified, are conventional methods and are carried out in accordance with the techniques or conditions described in the literatures in the art or in accordance with the product instructions. The materials, reagents, instruments, etc. used in the following examples, unless otherwise specified, may be obtained commercially. In the quantitative test in the following examples, three replicate experiments were set, and the results were averaged. In the following examples, unless otherwise specified, a first position of each nucleotide sequence in the sequence listing was a 5'-end nucleotide of corresponding DNA/RNA, and a last position is a 3'-end nucleotide of the corresponding DNA/RNA.

A pEC-H10-mCherry vector was recorded in the Chinese patent application (publication No.: CN 112980867A) with application number of 202110256579.8, and its preparation method was as follows:
Primers were designed and synthesized for the construction of the pEC-H10-mCherry vector by taking a strongest promoter H10 (GCTCAACCCTTACCGGTCGGCTCTAAGCCGGCGGCGTATGGTAAGTAAGCTCTGTTAT GTATAGTCCGAGCACGGCGAAAGGATACTC (SEQ ID No. 25)) reported by Wei et al. (Promoter library-based module combination (PLMC) technology for optimization of threonine biosynthesis in Corynebacterium glutamicum. Applied Microbiology and Biotechnology.2018(102)4117-4130.) as a template, and with a gene sequence of a mCherry protein and a sequence of an expression vector pEC-XK99E in Corynebacterium glutamicum. Primers was designed as follows (synthesized by Guangzhou GENEWIZ Company):
primer 1: 5'-GGATCTAGAGTCGACCTGCAG-3' (SEQ ID No. 26);
primer 2: 5'-TTAACTAGTATTGCGTTGCGCTCAC-3' (SEQ ID No. 27);
primer 3: 5'-CAATACTAGTTAATGTGAGTTAGCGCG-3' (SEQ ID No. 28);
primer 4: 
5′-AGAGCTTACCATACGCCGCCGGCTTAGAGCCGACCGGTAAGGGTTGAGCCTAGAGG
ATCCCCGGGTAC-3′ (SEQ ID No. 29);
primer 5: 5'-GCGTATGGTAAGCTCTGTTATGTTATGTATAGTCCGAGCACGGCGAAAGGATACTCAT GCGTAAAGGAGAAGAAG-3' (SEQ ID No. 30); and
primer 6: 5'-CGACTCTAGATCCGCCAAAACAGCC-3' (SEQ ID No. 31).

Construction method: a backbone region of a pEC plasmid (6743 bp) was amplified with the primer 1 and the primer 2 by taking pEC-XK99E as a template; a fragment (387 bp) containing an upstream region of a H10 promoter was amplified with the primer 3 and the primer 4 by taking the H10 promoter as a template; and a fragment (810 bp) containing a downstream region of the H10 promoter as well as a mCherry gene was amplified with the primer 5 and the primer 6 by taking a plasmid pBblactam containing the mCherry gene (Zhang et al., Development of a transcription factor based lactam biosensor. ACS synthetic biology.2017,6,439-445.) as a template.

At the end of PCR amplification, bands with lengths of 387 bp, 810 bp and 6743 bp were obtained respectively, and the above three bands were subjected to gel extraction by agarose gel electrophoresis; and after extraction, fusion PCR for amplification was performed with the primer 3 and the primer 6 by taking DNA fragments of the bands with lengths of 387 bp and 810 bp as templates. The resulting PCR amplified fragments were subjected to gel extraction. Then, the extracted fragments (i.e., fragments containing H10 and mCherry) and pEC backbone fragments were respectively enzymatically digested (SpeI, XbaI), and the fragments were purified with a column after enzymatic digestion. The pEC backbone fragments were mixed with the fragments containing H10 and mCherry at a molar ratio of 1: 2, and a T4 DNA ligase reaction solution was added. The fragments were transferred to an E. coli DH5α strain after ligation at 16°C for 1 h. The resulting recombinant vector with a correct sequence was the pEC-H10-mCherry vector.

pk18 vector: Addgene, Cat. No. MLCC1103.

Corynebacterium glutamicum CGMCC No. 12856 (strain number: YP097158) in the following example had been preserved in the General Microbiological Center of the China Microbiological Culture Collection Management Committee on August 16, 2016, with the preservation number of CGMCC No. 12856.

Corynebacterium glutamicum CGMCC No. 21220 (strain number: YPGLU001) in the following example had been preserved in the General Microbiological Center of the China Microbiological Culture Collection Management Committee on November 23, 2020, with the preservation number of CGMCC No. 21220.

Corynebacterium glutamicum CGMCC No. 21260 (strain number: YPFV1) in the following example had been preserved in the General Microbiological Center of the China Microbiological Culture Collection Management Committee on November 30, 2020, with the preservation number of CGMCC No. 21260.

### Example 1: EP6 had promoter activity.

### I. A pEC-PyfjB-mCherry vector was constructed to promote the expression of mCherry with a strong promoter PyfjB in E. coli.

By taking the pEC-H10-mCherry vector as a backbone, the pEC-PyfjB-mCherry vector containing a W3110yfjB promoter (SEQ ID No. 1: gaatttetecgcgtttttttcgcattcatctcgetaacttcgettattatggggatcagtttcagggtttcaagggaageactcacattgtcatcaatet tcgcaacaaggacctcggaaaa) in E. coli was constructed by using a NEBuilder recombination technology; the promotion intensity of PyfjB was detected according to the expression of a reporter protein mCherry, and treated as a control for subsequent mutant promoter sequences. The primer sequences were as follows (synthesized by Shanghai Invitrogen Company):
pEC01: 5'-ATGCGTAAAGGAGAAGAAGATAAC-3'(SEQ ID No. 32);
pEC02: 5'-CTAGAGGATCCCCGGGTAC-3'(SEQ ID No. 33);
P1:
   5'- CATGGAATTCGAGCTCGGTACCCGGGGATCCTCTAGGAATTTCTCCGCGTTTTTTT-3'( SEQ ID No. 34); and
P2:

5′- AATGATAGCCATGTTATCTTCTTCTCCTTTACGCATTTTTCCGAGGTCCTTGTTGC-3′(S
EQ ID No. 35).

Construction method: a backbone region (7772 bp) of the pEC-H10-mCherry vector was amplified with primers pEC01 and pEC02 by taking the pEC-H10-mCherry vector as a template; and a fragment (194 bp) of the yfjB promoter (abbreviated as PyfjB) containing a homologous arm of the pEC-H10-mCherry vector was amplified with primers P1 and P2 by taking the DNA fragment shown in SEQ ID No. 1 as a template.

The above two DNA fragments (the backbone region of the pEC-H10-mCherry vector and the PyfjB fragment containing the homologous arm of the pEC-H10-mCherry vector) were separated and purified by agarose gel electrophoresis, and then ligated with a NEBuilder enzyme (NEB) at 50°C for 30 min; monoclones grown after the transformation of the ligation product into E. coli DH5a were identified with primers pECF/R (pECF: 5'-GTACCCGGGGATCCTCTAG-3' (SEQ ID No. 36), pECR: 5'-GTTATCTTCTTCTCCTTTACGCAT-3' (SEQ ID No. 37)); and plasmids were extracted to obtain a positive recombinant vector with a correct sequence, i.e., pEC-PyfjB-mCherry.

A sequence of the recombinant vector pEC-PyfjB-mCherry was SEQ ID No. 2 in the sequence listing, in which the yfjB promoter promotes the expression of the reporter gene mCherry, and the intensity of the yfjB promoter was obtained by detecting a fluorescence value of mCherry.

### II. An expression vector pEC-EP6-mCherry for a mutant yfjB promoter was constructed.

For a PyfjB mutation shown in SEQ ID No. 1, the EP6 promoter was obtained with a size of 124 bp and a sequence of SEQ ID No. 3.

The vector was constructed by using the NEBuilder recombination technology, and the primers were designed as follows (synthesized by Shanghai Invitrogen Company):
P5:
   5'-CATGGAATTCGAGCTCGGTACCCGGGGATCCTCTAGGAATTTCTTCGCGTTTTTTT-3'( SEQ ID No. 38); and
P6:

5′- AATGATAGCCATGTTATCTTCTTCTCCTTTACGCATGATTTGGAGGTCCCTGCGTT-3′(S
EQ ID No. 39).

Construction method: a backbone region (7772 bp) of the pEC-H10-mCherry vector was amplified with primers pEC01 and pEC02 by taking the pEC-PyfjB-mCherry vector as a template; and a fragment (196 bp) of the EP6 promoter containing a homologous arm of the pEC-H10-mCherry vector was amplified with the primers P5 and P6 by taking a DNA fragment shown in SEQ ID No. 3 as a template.

The above two DNA fragments (the backbone region of the pEC-H10-mCherry vector and the EP6 promoter fragment containing the homologous arm of the pEC-H10-mCherry vector) were separated and purified by agarose gel electrophoresis, and then ligated with a NEBuilder enzyme (NEB) at 50°C for 30 min; monoclones grown after the transformation of the ligation product into E. coli DH5a was identified with primers pECF/R (pECF: 5'-GTACCCGGGGATCCTCTAG-3' (SEQ ID No. 36), pECR: 5'-GTTATCTTCTTCTCCTTTACGCAT-3' (SEQ ID No. 37)); and plasmids were extracted to obtain a positive recombinant vector with a correct sequence, marked as pEC-EP6-mCherry.

pEC-EP6-mCherry was a recombinant vector obtained by replacing PyfjB (SEQ ID No. 1) of pEC-PyfjB-mCherry with EP6 (SEQ ID No. 3).

### III. Determination of promoter activity.

The pEC-EP6-mCherry vector in step II and the pEC-PyfjB-mCherry vector in step I were introduced into wild-type Corynebacterium glutamicum ATCC13032 respectively to obtain recombinant bacteria ATCC13032/pEC-EP6-mCherry and ATCC13032/pEC-PyfjB-mCherry; the pEC-EP6-mCherry in step II and the pEC-PyfjB-mCherry vector in step I were introduced into the wild-type Corynebacterium glutamicum ATCC13869 respectively to obtain recombinant bacteria ATCC13869/pEC-EP6-mCherry and ATCC13869/pEC-PyfjB-mCherry; and the pEC-EP6-mCherry in step II and the pEC-PyfjB-mCherry vector in step I were introduced into the wild-type Corynebacterium glutamicum ATCC14067 respectively to obtain recombinant bacteria ATCC14067/pEC-EP6-mCherry and ATCC14067/pEC-PyfjB-mCherry. The promotion intensities of these two promoters in Corynebacterium glutamicum were compared.

Monoclones of the above 6 recombinant bacteria were respectively inoculated into a 96 deep-well plate containing 900 µL of LBHIS medium, wherein each monoclone contained 3 replicates, a fluorescence value of mCherry was detected by a microplate reader after culture at 30°C and 800 rpm for 24 h, and the promoter activities of yfjB and EP6 were compared according to the fluorescence intensities.

LBHIS medium: 5 g of Tryptone/L; 5 g of NaCl/L; 2.5 g of yeast extract/L; 18.5 g of brain heart infusion (BHI)/L; and 91 g of sorbitol/L, with pH of 7.2.

The results showed that the EP6 promoter had promoter activity which was significantly higher than that of the yfjB promoter (FIG 1).

### Example 2: Replacement of lysA gene promoter in lysine metabolism pathway of Corynebacterium glutamicum and its effect on lysine yield.

### I. Preparation of recombinant bacteria.

The EP6 and yfjB promoters were replaced with an original promoter (positions 1-148 of SEQ ID No. 4) of a key gene lysA of a lysine metabolism pathway of Corynebacterium glutamicum CGMCC No. 12856 with high-yield lysine by using a pk18 vector and a homologous recombination method, so as to promote the expression of lysA in the lysine metabolism pathway by using an exogenous promoter, thereby obtaining lysine-producing bacteria with an exogenous promoter-gene combination. In the fermentation test of these production strains, it was found that the strain with EP6 promoting lysA could increase the yield of lysine, and this strain was named YPL-4-042. Positions 149-1486 of SEQ ID No. 4 were a sequence of lysA.

A vector was constructed by using the NEBuilder recombination technology, and the primers were designed as follows (synthesized by Shanghai Invitrogen Company):
P7: 
5′- CAGTGCCAAGCTTGCATGCCTGCAGGTCGACTCTAGCCCAGGTGAAGAAGTCGTTG
-3′(SEQ ID No. 40);
P8: 5'-AAAAAAACGCGGAGAAATTCATGCCCCGTTCGACAATAAA-3'(SEQ ID No. 41);
P9: 5'-TTTATTGTCGAACGGGGCATGAATTTCTCCGCGTTTTTTT-3'(SEQ ID No. 42);
P10: 5'-GCGAGATCAGCTGGTGTCATTTTTCCGAGGTCCTTGTTGC-3'(SEQ ID No. 43);
P11: 5'-GCAACAAGGACCTCGGAAAAATGACACCAGCTGATCTCGC-3'(SEQ ID No. 44);
P12: 
5′- CAGCTATGACCATGATTACGAATTCGAGCTCGGTACCCTCGAACATCATCTCCACGC
C-3′(SEQ ID No. 45);
P8E: 5'-AAAAAAACGCGAAGAAATTCATGCCCCGTTCGACAATAAA-3'(SEQ ID No. 46);
P9E: 5'-TTTATTGTCGAACGGGGCATGAATTTCTTCGCGTTTTTTT-3'(SEQ ID No. 47);
P10E: 5'-GCGAGATCAGCTGGTGTCATGATTTGGAGGTCCCTGCGTT-3'(SEQ ID No. 48); and
P11E: 5'-AACGCAGGGACCTCCAAATCATGACACCAGCTGATCTCGC-3'(SEQ ID No. 49).

The specific methods for homologous recombination were as follows:
PCR amplification was performed by taking Corynebacterium glutamicum ATCC13032 as a template and with primers P7/P8 (a PCR product sequence was positions 1-801 of SEQ ID No. 5) to obtain an upstream homologous arm fragment (801 bp); PCR amplification was performed by taking the vector pEC-PyfjB-mCherry as a template and with primers P9/P10 to obtain PyfjB (162 bp) (a PCR product sequence was positions 762-923 of SEQ ID No. 5); and PCR amplification was performed by taking Corynebacterium glutamicum ATCC13032 as a template and with primers P11/P12 (a PCR product sequence was positions 884-1648 of SEQ ID No. 5) to obtain a downstream homologous arm fragment (765 bp).

After the PCR reaction, the three PCR products were recovered by electrophoresis by using a column-type DNA gel recovery kit. The three recovered PCR products were ligated with a pK18mobsacB vector (Addgene, this vector contained kanamycin resistance as a selection marker), which is purified after XbalI and BamHI enzymatic digestion, at 50°C for 30 min by using a NEBuilder enzyme (NEB). Monoclones grown after the transformation of the ligation product into E. coli DH5a were subjected to PCR identification with primers M13 (M13F: 5'-TGTAAAACGACGGCCAGT-3'(SEQ ID No. 50), M13R: 5'-CAGGAAACAGCTATGACC-3'(SEQ ID No. 51)) to obtain a positive integrated vector (recombinant vector). A recombinant vector with a correct sequence was extracted and marked as pK18-PyfjB-lysAOE. This recombinant vector contained a kanamycin resistance marker, and a recombinant in which the vector was integrated into a genome was obtained by kanamycin screening.

pK18-PyfjB-lysAOE contained a DNA fragment as shown in SEQ ID No. 5, in which positions 1-801 were a sequence of an upstream homologous arm fragment, positions 884-1648 were a sequence of a downstream homologous arm fragment, and positions 762-923 were a sequence of PyfjB.

PCR amplification was performed by taking Corynebacterium glutamicum ATCC13032 as a template and with primers P7/P8E (positions 1-801 of SEQ ID No. 6) to obtain an upstream homologous arm fragment (801 bp); PCR amplification was performed by taking pEC-PyfjB-mCherry as a template and with primers P9E/P10E to obtain EP6 (164 bp) (positions 762-925 of SEQ ID No. 6); and PCR amplification was performed by taking Corynebacterium glutamicum ATCC13032 as a template and with primers P11E/P12 (positions 886-1650 of SEQ ID No. 6) to obtain a downstream homologous arm fragment (765 bp).

After the PCR reaction, the three PCR products were recovered by electrophoresis by using a column-type DNA gel recovery kit. The three recovered PCR products were ligated with a pK18mobsacB vector (Addgene, this vector contained kanamycin resistance as a selection marker), which is purified after XbalI and BamHI enzymatic digestion, at 50°C for 30 min by using a NEBuilder enzyme (NEB). Monoclones grown after the transformation of the ligation product into E. coli DH5a were subjected to PCR identification with primers M13 (M13F: 5'-TGTAAAACGACGGCCAGT-3'(SEQ ID No. 50), M13R: 5'-CAGGAAACAGCTATGACC-3'(SEQ ID No. 51)) to obtain a positive integrated vector (recombinant vector). A recombinant vector with a correct sequence was extracted and marked as pK18-EP6-lysAOE. This recombinant vector contained a kanamycin resistance marker, and a recombinant in which the vector was integrated into a genome was obtained by kanamycin screening.

pK18-EP6-lysAOE contained a DNA fragment as shown in SEQ ID No. 6, in which positions 1-801 were a sequence of an upstream homologous arm fragment, positions 886-1650 were a sequence of a downstream homologous arm fragment, and positions 762-925 were a sequence of EP6.

The recombinant vectors pK18-PyfjB-lysAOE and pK18-EP6-lysAOE with correct sequencing were respectively electrotransformed into Corynebacterium glutamicum CGMCC No. 12856 with high-yield lysine, and cultured in a recovery medium according to rejuvenation culture conditions. Single colonies generated by culture were subjected to PCR identification with P13/P14 primers. The recombinant bacteria introduced into pK18-PyfjB-lysAOE were subjected to PCR amplification to obtain a fragment (885 bp) (SEQ ID No. 7), which was a positive strain and marked as CGMCC No.12856/pK18-PyfjB-lysAOE. The recombinant bacteria introduced into pK18-EP6-lysAOE were subjected to PCR amplification to obtain a fragment (885 bp) (SEQ ID No. 8), which was a positive strain and marked as CGMCC No.12856/pK18-EP6-lysAOE. Bacteria that cannot amplify any fragment were original bacteria.

The positive strain was cultured on a medium containing 15% sucrose, and single colonies produced by culture were further subjected to PCR amplification with P15/P16 primers; a strain with a size of 841 bp (SEQ ID No. 9) was amplified as a positive strain with PyfjB replacing an original lysA promoter of Corynebacterium glutamicum CGMCC No.12856 with high-yield lysine. A strain with a size of 843 bp (SEQ ID No. 10) was amplified as a positive strain with EP6 replacing the original lysA promoter of Corynebacterium glutamicum CGMCC No. 12856 with high-yield lysine. Bacteria that cannot amplify any fragment were original bacteria.

The positive strains obtained from the recombinant bacteria introduced into pK18-PyfjB-lysAOE and pK18-EP6-lysAOE were named YPL-4-041 (a PyfjB-lysA integrated strain) and YPL-4-042 (an EP6-lysA integrated strain), respectively.

YPL-4-041 (PyfjB-lysA integrated strain) was recombinant bacteria obtained by replacing the original lysA promoter (positions 1-148 of SEQ ID No. 4) of Corynebacterium glutamicum CGMCC No. 12856 with high-yield lysine by a yfjB promoter and keeping the other nucleotides in its genome unchanged, which can increase the yield of lysine. YPL-4-042 (EP6-lysA integrated strain) was recombinant bacteria obtained by replacing the original lysA promoter (positions 1-148 of SEQ ID No. 4) of Corynebacterium glutamicum CGMCC No. 12856 with high-yield lysine by an EP6 promoter and keeping the other nucleotides in its genome unchanged. The EP6 promoter, as a mutant yfjB promoter, promotes the expression of lysA and can improve the yield of lysine.

The primers for PCR identification were as follows:
P13: 5'-GGCGACGATCTTGTTTGACC-3' (inside cg1332 gene) (SEQ ID No. 52);
P14: 5'-ACTGATCCCCATAATAAGCG-3' (inside PyfjB) (SEQ ID No. 53);
P15: 5'-CGCTTATTATGGGGATCAGT-3' (inside PyfjB) (SEQ ID No. 54); and
P16: 5'-GTAGTAGACATCGAAATCGG-3' (inside argS gene) (SEQ ID No. 55).

Recovery medium: a solvent was water, and a solute and its content in the recovery medium were 5 g/L glucose, 3 g/L urea, 10 g/L yeast powder, 10 g/L biotin, 15 g/L soybean meal powder, 0.5 g/L succinic acid, 1 g/L potassium dihydrogen phosphate, 2.5 g/L sodium chloride, 91 g/L sorbitol, and 18.5 g/L brain heart infusion, respectively, with pH of 7.0.

Culture medium containing 15% sucrose: a solvent was water, and a solute and its content in the culture medium containing 15% sucrose were 15 g/L sucrose, 3 g/L urea, 10 g/L yeast powder, 10 g/L biotin, 15 g/L soybean meal powder, 0.5 g/L succinic acid, 1 g/L potassium dihydrogen phosphate, 2.5 g/L sodium chloride, 91 g/L sorbitol, 18.5 g/L brain heart infusion, and 15 g/L of agar powder, respectively, with pH of 7.0.

Rejuvenation culture conditions: cells were resuspended in the recovery medium at 46°C, transferred to an EP tube, cultured for 45-60 min in a 220 rpm, 37°C shaker, and then transferred to a 30°C shaker for 45-60 min at the same speed.

### II. L-lysine fermentation experiment.

The above strains YPL-4-041 and YPL-4-042 and an original strain Corynebacterium glutamicum CGMCC No. 12856 were fermented in a fermentation tank of BLBIO-5GC-4-H model (Shanghai Bailun Biotechnology Co., Ltd.) with a fermentation medium 1 and a culture condition 1, the L-lysine yield was detected at the end of fermentation, and each strain was repeated three times.

The yield of L-lysine was detected by high-performance liquid chromatography.

Fermentation medium 1: a solvent was water, and a solute and its content in the fermentation medium 1 were 30 g/L starch hydrolyzed sugar, 12 g/L ammonium sulfate, 0.87 g/L magnesium sulfate, 20 g/L molasses, 3 mL/L acidified corn syrup, 0.4 mL/L phosphoric acid, 0.53 g/L potassium chloride, 4 mL/L defoamer (2% Paodi), 120 mg/L ferrous sulfate, 120 mg/L manganese sulfate, 42 mg/L niacinamide, 6.3 mg/L calcium pantothenate, 6.3 mg/L vitamin B1, 0.6 g/L copper sulfate, 0.6 g/L zinc sulfate, and 0.88 mg/L biotin, respectively. The acidified corn syrup was a product from Ningxia EPPEN Biotechnology Co., Ltd.

Culture condition 1:
corrected DO of 100%, temperature of 37°C, air volume of 4 L/min, speed of 1000 rpm, tank pressure of 0 Mpa, and calibrated after 5min;
inoculation amount: 10%;
culture temperature: 37°C;
pH: 6.9±0.05;
dissolved oxygen DO: 10-30%;
initial conditions: temperature of 37°C, pH of 6.9, tank pressure of 0 Mpa, air volume of 3 L/min, and speed of 550 rpm;
whole-process control: whole-process control 1, the dissolved oxygen < 30%, and the speed was sequentially increased by 750 rpm→800 rpm→ air volume of 4 L/min→850 rpm→950 rpm; 2, the tank pressure was increased by 0.01 Mpa after 6 h of fermentation, and the tank pressure was increased by 0.02 Mpa→0.03 Mpa→ 0.04 Mpa→0.05 Mpa after 12 h of fermentation;
residual sugar control: 0.1-0.2% before F12h; 0.1-0.05% after F12h in combination with DO requirements;
ammonia-nitrogen control: 0.1-0.15 before F12h; 0.15-0.25 for F12-F32h; 0.1-0.15 after F32h; material fed-batch: 25% of ammonia, 70% of concentrated sugar, 50% of ammonium sulfate, and 10% of Paodi; and
fermentation cycle: about 48 h.

The results showed that the L-lysine yield of the strain CGMCC No. 12856 was 18.9 g/100 mL; the L-lysine yield of YPL-4-041 was 19.2 g/100 mL, which was significantly higher than that of the strain CGMCC No. 12856 (P<0.05); and the L-lysine yield of YPL-4-042 was 19.7 g/100 mL, which was significantly higher than that of the strain CGMCC No. 12856 (P<0.05). It was indicated that replacing the original lysA promoter with the yfjB or EP6 promoter in lysine-producing bacteria CGMCC No. 12856 could increase the yield of lysine, which was higher as the promotion of the expression of lysA by the mutated EP6.

### Example 3: increase of BBD29_14295 gene promoter in glutamic acid metabolism pathway in Corynebacterium glutamicum and its effect on glutamic acid yield.

### I. Preparation of recombinant bacteria.

The EP6 and yfjB promoters were replaced with an original promoter (positions 1-36 of SEQ ID No. 11) of a key gene BBD29_14295 of a glutamic acid metabolism pathway of Corynebacterium glutamicum CGMCC No. 21220 with high-yield glutamic acid by using a pk18 vector and a homologous recombination method, so as to promote the expression of BBD29_14295 in the glutamic acid metabolism pathway by using an exogenous promoter, thereby obtaining glutamic acid-producing bacteria with an exogenous promoter-gene combination. In the fermentation test of these production strains, it was found that the strain with EP6 promoting BBD29_14295 could increase the yield of glutamic acid, and this strain was named YPG-092. Positions 37-1161 of SEQ ID No. 11 were a sequence of BBD29_14295.

A vector was constructed by using the NEBuilder recombination technology, and the primers were designed as follows (synthesized by Shanghai Invitrogen Company):
P17: 
5′- CAGTGCCAAGCTTGCATGCCTGCAGGTCGACTCTAGCGGAAACAACGCAGCCATAG
-3′(SEQ ID No. 56);
P18: 5'-AAAAAAACGCGGAGAAATTCTTAGAGGAATTTAACGCCTT-3'(SEQ ID No. 57);
P19: 5'-AAGGCGTTAAATTCCTCTAAGAATTTCTCCGCGTTTTTTT-3'(SEQ IDNo. 58);
P20: 5'-TCGTCGATTAGCTCGAACATTTTTCCGAGGTCCTTGTTGC-3'(SEQ ID No. 59);
P21: 5'-GCAACAAGGACCTCGGAAAAATGTTCGAGCTAATCGACGA-3'(SEQ ID No. 60);
P22: 
5′- CAGCTATGACCATGATTACGAATTCGAGCTCGGTACCCATGACGTCCTGGAGCGCTG
C-3′(SEQ ID No. 61);
P18E: 5'-AAAAAAACGCGAAGAAATTCTTAGAGGAATTTAACGCCTT-3'(SEQ ID No. 62);
P19E: 5'-AAGGCGTTAAATTCCTCTAAGAATTTCTTCGCGTTTTTTT-3'(SEQ ID No. 63);
P20E: 5'-TCGTCGATTAGCTCGAACATGATTTGGAGGTCCCTGCGTT-3'(SEQ ID No. 64); and
P21E: 5'-AACGCAGGGACCTCCAAATCATGTTCGAGCTAATCGACGA-3'(SEQ ID No. 65).

The specific methods for homologous recombination were as follows:
PCR amplification was performed by taking Corynebacterium glutamicum ATCC13869 as a template and with primers P17/P18 (positions 1-792 of SEQ ID No. 12) to obtain an upstream homologous arm fragment (792 bp); PCR amplification was performed by taking pEC-PyfjB-mCherry as a template and with primers P19/P20 to obtain PyfjB (162 bp) (positions 753-914 of SEQ ID No. 12); and PCR amplification was performed by taking Corynebacterium glutamicum ATCC13869 as a template and with primers P21/P22 (positions 875-1699 of SEQ ID No. 12) to obtain a downstream homologous arm fragment (825 bp).

After the PCR reaction, the three PCR products were recovered by electrophoresis by using a column-type DNA gel recovery kit. The three recovered PCR products were ligated with a pK18mobsacB vector (Addgene, this vector contained kanamycin resistance as a selection marker), which was purified after XbalI and BamHI enzymatic digestion, at 50°C for 30 min by using a NEBuilder enzyme (NEB). Monoclones grown after the transformation of the ligation product into E. coli DH5a were subjected to PCR identification with M13 primers (M13F: 5'-TGTAAAACGACGGCCAGT-3'(SEQ ID No. 50), M13R: 5'-CAGGAAACAGCTATGACC-3'(SEQ ID No. 51)) to obtain a positive integrated vector (recombinant vector). A recombinant vector with a correct sequence was marked as pK18-Py1jB-BBD29_14295OE. This recombinant vector contained a kanamycin resistance marker, and a recombinant in which the vector was integrated into a genome was obtained by kanamycin screening.

pK18-PyfjB-BBD29_14295OE contained a DNA fragment as shown in SEQ ID No. 12, in which positions 1-792 were a sequence of an upstream homologous arm fragment, positions 875-1699 were a sequence of a downstream homologous arm fragment, and positions 753-914 were a sequence of PyfjB.

PCR amplification was performed by taking Corynebacterium glutamicum ATCC13869 as a template and with primers P17/P18E (positions 1-792 of SEQ ID No. 13) to obtain an upstream homologous arm fragment (792 bp); PCR amplification was performed by taking the vector pEC-EP6-mCherry as a template and with primers P19E/P20E to obtain EP6 (164 bp) (positions 753-916 of SEQ ID No. 13); and PCR amplification was performed by taking Corynebacterium glutamicum ATCC13869 as a template and with primers P21E/P22 (positions 877-1701 of SEQ ID No. 13) to obtain a downstream homologous arm fragment (825 bp).

After the PCR reaction, the three PCR products were recovered by electrophoresis by using a column-type DNA gel recovery kit. The three recovered PCR products were ligated with a pK18mobsacB vector (Addgene, this vector contained kanamycin resistance as a selection marker), which was purified after XbalI and BamHI enzymatic digestion, at 50°C for 30 min by using a NEBuilder enzyme (NEB). Monoclones grown after the transformation of the ligation product into E. coli DH5a were subjected to PCR identification with M13 primers (M13F: 5'-TGTAAAACGACGGCCAGT-3'(SEQ ID No. 50), M13R: 5'-CAGGAAACAGCTATGACC-3'(SEQ ID No. 51)). A recombinant vector with a correct sequence was marked as pK18-EP6-BBD29_14295OE. This recombinant vector contained a kanamycin resistance marker, and a recombinant in which the vector was integrated into a genome was obtained by kanamycin screening.

pK18-EP6-BBD29_14295OE contained a DNA fragment as shown in SEQ ID No. 13, in which positions 1-792 were a sequence of an upstream homologous arm fragment, positions 877-1701 were a sequence of a downstream homologous arm fragment, and positions 753-916 were a sequence of EP6.

The recombinant vectors (pK18-PyfjB-BBD29_14295OE and pK18-EP6-BBD29_14295OE) with correct sequencing were respectively electrotransformed into Corynebacterium glutamicum CGMCC No. 21220 with high-yield glutamic acid, and cultured in a recovery medium (same as Example 2) according to rejuvenation culture conditions (same as Example 2). Single colonies generated by culture were subjected to PCR identification with P23/P24 primers. A fragment (849 bp) (SEQ ID No. 14) obtained by PCR amplification of PyfjB-BBD29_14295 integrated bacteria was a positive strain, and a fragment (849 bp) (SEQ ID No. 15) obtained by PCR amplification of EP6-BBD29_14290 integrated bacteria was a positive strain. Bacteria that cannot amplify any fragment were original bacteria.

The positive strain was cultured on a medium containing 15% sucrose (same as the medium containing 15% sucrose in Example 2). Single colonies produced by culture were further subjected to PCR amplification with P25/P26 primers. A strain with a size of 889 bp (SEQ ID No. 16) was amplified as a strain with PyfjB replacing an original BBD29_14295 promoter on a Corynebacterium glutamicum CGMCC No. 21220 genome with high-yield glutamic acid. A strain with a size of 891 bp (SEQ ID No. 17) was amplified as a strain with EP6 replacing the original BBD29_14295 promoter on the Corynebacterium glutamicum CGMCC No. 21220 genome with high-yield glutamic acid. Bacteria that cannot amplify any fragment were original bacteria.

The recombinant strain obtained by replacing PyfjB with the original BBD29_14295 promoter was named YPG-091 (PyfjB-BBD29_14295 integrated strain), and the recombinant strain obtained by replacing EP6 with the original BBD29_14295 promoter was named YPG-092 (EP6-BBD29_14295 integrated strain).

YPG-091 (PyfjB-BBD29_14295 integrated strain) was recombinant bacteria obtained by replacing the original BBD29_14295 promoter (positions 1-36 of SEQ ID No. 11) of Corynebacterium glutamicum CGMCC No. 21220 with high-yield glutamic acid by PyfjB and keeping the other nucleotides in its genome unchanged, which can increase the yield of glutamic acid. YPG-092 (EP6-BBD29_14295 integrated strain) was recombinant bacteria obtained by replacing the original BBD29_14295 promoter (positions 1-36 of SEQ ID No. 11) of Corynebacterium glutamicum CGMCC No. 21220 with high-yield glutamic acid by EP6 and keeping the other nucleotides in its genome unchanged. The EP6 promoter, as a mutant yfjB promoter, promotes the expression of BBD29_14295 and can improve the yield of glutamic acid.

The primers for PCR identification were as follows:
P23: 5'-GAAGTAGGTC CTCGTGTTGC-3' (inside BBD29_14290 gene) (SEQ ID No. 66);
P24: 5'-ACTGATCCCC ATAATAAGCG-3' (inside PyfjB) (SEQ ID No. 67);
P25: 5'-CGCTTATTAT GGGGATCAGT-3' (inside PyfjB) (SEQ ID No. 68); and
P26: 5'-GACGAGTTGT GCTTGTAGTC-3'(inside BBD29_14295gene )(SEQ ID No. 69).

### II. L-glutamic acid fermentation experiment.

The above strains YPG-091 and YPG-092 and an original strain CGMCC No. 21220 were fermented in a fermentation tank of BLBIO-5GC-4-H model (Shanghai Bailun Biotechnology Co., Ltd.) with a fermentation medium 2 and a culture condition 2, and the L-glutamic acid yield and the OD value were detected at the end of fermentation. Each strain was repeated three times
The glutamic acid yield was detected by a biosensor.

Fermentation medium 2: a solvent was water, and a solute and its concentration in the fermentation medium 2 were 5.0 g/L glucose, 0.38 g/L phosphoric acid, 1.85 g/L magnesium sulfate, 1.6 g/L potassium chloride, 550 µg/L biotin, 300 µg/L vitamin B1, 10 mg/L ferrous sulfate, 10 g/dl manganese sulfate, 2.8 g/L KH₂PO₄, 0.75 mg/L vitamin C, 2.5 µg/L vitamin B12, 0.75 mg/L para-aminobenzoic acid, 0.0015 mL/dL antifoam, 1.5 g/L betaine, 7 mL/L sugarcane molasses, 77 mL/L corn syrup, and 1.7 g/L aspartic acid.

Culture condition 2:
corrected DO of 100%, temperature of 32.5°C, air volume of 7 L/min, speed of 700 rpm, tank pressure of 0 Mpa, and calibrated after 5 min;
inoculation amount: 13%; 0 h: speed of 400 rpm, air volume of 3 L/min, pressure of 0.05 MPA, and culture temperature of 32.5°C;
bacterium concentration OD=1.0: speed of 600 rpm, air volume of 5 L/min, pressure of 0.08 MPA, and culture temperature of 37°C;
bacterium concentration OD=1.0-bacterium concentration OD=1.4: speed of 700 rpm, air volume of 7 L/min, pressure of 0.11 MPA, and culture temperature of 38°C;
after 32 h-34 h of culture, the fermentation was completed, and 50-20% dissolved oxygen was used in the control process as a standard for increasing and decreasing the air volume;
pH: 7.0 for 0 h, 6.8 for 14 h; and
sugar fed-batch control: the concentration of sugar fed-batch in the fermentation tank was 50-55%, and the residual sugar in the fermentation tank was controlled to 0.5-1.0%.

The results showed that the L-glutamic acid yield of CGMCC No. 21220 was 102.1 g/L, and OD (562 nm) was 46.1; the L-glutamic acid yield of YPG-091 was 103.3 g/L, which was significantly higher than that of the strain CGMCC No. 21220 (P<0.05), and OD (562 nm) was 46.6; and the L-glutamic acid yield of YPG-092 was 105.1 g/L, which was significantly higher than that of the strain CGMCC No. 21220 (P<0.05), and OD (562 nm) was 47.1. It was indicated that replacing the original BBD29_14295 promoter with the yfjB or EP6 promoter in glutamic acid-producing bacteria CGMCC No. 21220 could increase the yield of glutamic acid, which was higher as the promotion of the expression of BBD29_14295 by the mutated EP6.

### Example 4: increase of ilvC gene promoter in glutamic acid metabolism pathway of Corynebacterium glutamicum and its effect on valine yield.

### I. Preparation of recombinant bacteria.

The EP6 and yfjB promoters were replaced with an original promoter (positions 1-179 of SEQ ID No. 18) of a key gene ilvC of a valine metabolism pathway of Corynebacterium glutamicum CGMCC No. 21260 with high-yield valine by using a pk18 vector and a homologous recombination method, so as to promote the expression of the ilvC gene in the glutamic acid metabolism pathway by using an exogenous promoter, thereby obtaining valine-producing bacteria with an exogenous promoter-gene combination. In the fermentation test of these production strains, it was found that the strain with EP6 promoting ilvC could increase the yield of valine, and this strain was named YPV-098. Positions 180-1196 of SEQ ID No. 18 were a sequence of ilvC.

A vector was constructed by using the NEBuilder recombination technology, and the primers were designed as follows (synthesized by Shanghai Invitrogen Company):
P27: 
5′- CAGTGCCAAGCTTGCATGCCTGCAGGTCGACTCTAGAGAGATCAACGACCGCCCAG
-3′(SEQ ID No. 70);
P28: 5'-AAAAAAACGCGGAGAAATTCTTAGATCTTGGCCGGAGCCA-3'(SEQ ID No. 71);
P29: 5'-TGGCTCCGGCCAAGATCTAAGAATTTCTCCGCGTTTTTTT-3'(SEQ ID No. 72);
P30: 5'-TAAAGCAGTTCAATAGCCATTTTTCCGAGGTCCTTGTTGC-3'(SEQ ID No. 73);
P31: 5'-GCAACAAGGACCTCGGAAAAATGGCTATTGAACTGCTTTA-3'(SEQ ID No. 74);
P32: 5′- CAGCTATGACCATGATTACGAATTCGAGCTCGGTACCCTGAGGCGCTTGGTGAAGGT G-3′(SEQ ID No. 75);
P28E: 5'-AAAAAAACGCGAAGAAATTC TTAGATCTTGGCCGGAGCCA-3'(SEQ ID No. 76);
P29E: 5'-TGGCTCCGGCCAAGATCTAAGAATTTCTTCGCGTTTTTTT-3'(SEQ ID No. 77);
P30E: 5'-TAAAGCAGTTCAATAGCCATGATTTGGAGGTCCCTGCGTT-3'(SEQ ID No. 78); and
P31E: 5'-AACGCAGGGACCTCCAAATCATGGCTATTGAACTGCTTTA-3'(SEQ ID No. 79).

The specific methods for homologous recombination were as follows:
PCR amplification was performed by taking Corynebacterium glutamicum ATCC14067 as a template and with primers P27/P28 (positions 1-805 of SEQ ID No. 19) to obtain an upstream homologous arm fragment (805 bp); PCR amplification was performed by taking the vector pEC-PyfjB-mCherry as a template and with primers P29/P30 to obtain PyfjB (162 bp) (positions 766-927 of SEQ ID No. 19); and PCR amplification was performed by taking Corynebacterium glutamicum ATCC14067 as a template and with primers P31/P32 (positions 888-1822 of SEQ ID No. 19) to obtain a downstream homologous arm fragment (935 bp).

After the PCR reaction, the three PCR products were recovered by electrophoresis by using a column-type DNA gel recovery kit. The three recovered PCR products were ligated with a pK18mobsacB vector (Addgene, this vector contained kanamycin resistance as a selection marker), which was purified after XbalI and BamHI enzymatic digestion, at 50°C for 30 min by using a NEBuilder enzyme (NEB). Monoclones grown after the transformation of the ligation product into E. coli DH5a were subjected to PCR identification with M13 primers (M13F: 5'-TGTAAAACGACGGCCAGT-3'(SEQ ID No. 50), M13R: 5'-CAGGAAACAGCTATGACC-3'(SEQ ID No. 51)) to obtain a positive integrated vector (recombinant vector). A recombinant vector with a correct sequence was marked as pK18-PyfjB-ilvCOE. This recombinant sequence contained a kanamycin resistance marker, and a recombinant in which the vector was integrated into a genome was obtained by kanamycin screening.

pK18-PyfjB-ilvCOE contained a DNA fragment as shown in SEQ ID No. 19, in which positions 1-805 were a sequence of an upstream homologous arm fragment, positions 888-1822 were a sequence of a downstream homologous arm fragment, and positions 766-927 were a sequence of PyfjB.

PCR amplification was performed by taking Corynebacterium glutamicum ATCC14067 as a template and with primers P27/P28E (positions 1-805 of SEQ ID No. 20) to obtain an upstream homologous arm fragment (805 bp); PCR amplification was performed by taking the vector pEC-EP6-mCherry as a template and with primers P29E/P30E to obtain EP6 (164 bp) (positions 766-929 of SEQ ID No. 20); and PCR amplification was performed by taking Corynebacterium glutamicum ATCC14067 as a template and with primers P31E/P32 (positions 890-1824 of SEQ ID No. 20) to obtain a downstream homologous arm fragment (935 bp).

After the PCR reaction, the three PCR products were recovered by electrophoresis by using a column-type DNA gel recovery kit. The three recovered PCR products were ligated with a pK18mobsacB vector (Addgene, this vector contained kanamycin resistance as a selection marker), which was purified after XbalI and BamHI enzymatic digestion, at 50°C for 30 min by using a NEBuilder enzyme (NEB). Monoclones grown after the transformation of the ligation product into E. coli DH5a were subjected to PCR identification with M13 primers (M13F: 5'-TGTAAAACGACGGCCAGT-3'(SEQ ID No. 50), M13R: 5'-CAGGAAACAGCTATGACC-3'(SEQ ID No. 51)) to obtain a positive integrated vector (recombinant vector). A recombinant vector with a correct sequence was marked as pK18-EP6-ilvCOE. This recombinant vector contained a kanamycin resistance marker, and a recombinant in which the vector was integrated into a genome was obtained by kanamycin screening.

pK18-EP6-ilvCOE contained a DNA fragment as shown in SEQ ID No. 20, in which positions 1-805 were a sequence of an upstream homologous arm fragment, positions 890-1824 were a sequence of a downstream homologous arm fragment, and positions 766-929 were a sequence of EP6.

The integrated vectors (pK18-PyfjB-ilvCOE and pK18-EP6-ilvCOE) with correct sequencing were respectively electrotransformed into Corynebacterium glutamicum CGMCC No. 21260 with high-yield valine, and cultured in a recovery medium (same as Example 2) according to rejuvenation culture conditions (same as Example 2). Single colonies generated by culture were subjected to PCR identification with P33/P34 primers. A fragment (901 bp) (SEQ ID No. 21) obtained by PCR amplification of PyfjB-ilvC integrated bacteria was a positive strain, and a fragment (901 bp) (SEQ ID No. 22) obtained by PCR amplification of EP6-ilvC integrated strain was a positive strain. Bacteria that cannot amplify any fragment were original bacteria.

The positive strain was cultured on a medium containing 15% sucrose (same as the medium containing 15% sucrose in Example 2). Single colonies produced by culture were further subjected to PCR amplification with P35/P36 primers. A strain with a size of 1023 bp (SEQ ID No. 23) was amplified as a positive strain with PyfjB replacing an original ilivC promoter on a Corynebacterium glutamicum CGMCC No. 21260 genome with high-yield valine. A strain with a size of 1025 bp (SEQ ID No. 24) was amplified as a strain with EP6 replacing the original ilvC promoter on the Corynebacterium glutamicum CGMCC No.21260 genome with high-yield valine. Bacteria that cannot amplify any fragment were original bacteria.

The recombinant strain obtained by replacing PyfjB with the original ilvC promoter was named YPV-097 (PyfjB-ilvC integrated strain), and the recombinant strain obtained by replacing EP6 with the original ilvC promoter was named YPV-098 (EP6-ilvC integrated strain).

YPV-097 (PyfjB-ilvC integrated strain) was recombinant bacteria obtained by replacing the original ilvC promoter (positions 1-179 of SEQ ID No. 18) of Corynebacterium glutamicum CGMCC No. 21260 with high-yield valine with PyfjB and keeping the other nucleotides in its genome unchanged, which can increase the yield of valine. YPV-098 (EP6-ilvC integrated strain) was recombinant bacteria obtained by replacing the original ilvC promoter (positions 1-179 of SEQ ID No. 18) of Corynebacterium glutamicum CGMCC No. 21260 with high-yield valine with EP6 and keeping the other nucleotides in its genome unchanged. The EP6 promoter, as a mutant yfjB promoter, promotes the expression of ilvC and can improve the yield of valine.

The primers for PCR identification were as follows:
P33: 5'-CCCGACTTTG TTACCCTTTC-3' (inside CEY17_RS06885 gene) (SEQ ID No. 80);
P34: 5'-ACTGATCCCC ATAATAAGCG-3' (inside PyfjB) (SEQ ID No. 81);
P35: 5'-CGCTTATTAT GGGGATCAGT-3' (inside PyfjB) (SEQ ID No. 82); and
P36: 5'-GGGTGGTTGT TGTAGGAAGC-3' (inside ilvC gene) (SEQ ID No. 83).

### II. Valine fermentation experiment.

The above strains YPV-097 and YPV-098 and an original strain Corynebacterium glutamicum CGMCC No. 21260 were fermented in a fermentation tank of BLBIO-5GC-4-H model (Shanghai Bailun Biotechnology Co., Ltd.) with a fermentation medium 3 and a culture condition 3, the L-valine yield and the OD value were detected at the end of fermentation. Each strain was repeated three times

The yield of L-valine was detected by high-performance liquid chromatography.

Fermentation medium 3: a solvent was water, and a solute and its concentration were 14 g/L ammonium sulfate, 1 g/L potassium dihydrogen phosphate, 0.5 g/L magnesium sulfate, 2 g/L yeast powder, 18 mg/L ferrous sulfate, 4.2 mg/L manganese sulfate, 0.02 mg/L biotin, 2 mg/L vitamin B1, 0.5 mL/L antifoam (CB-442), and 40 g/L 70% glucose (base sugar), respectively.

Culture condition 3:
corrected DO of 100%, temperature of 33°C, air volume of 1 L/min, speed of 400 rpm, tank pressure of 0.01 Mpa, and calibrated after 5 min;
the inoculation amount was 3.5%, and the culture temperature was 33°C;
pH of 7.0±0.05; dissolved oxygen DO of 10-20%;
initial conditions: temperature of 33°C, pH of 7.0, tank pressure of 0 Mpa, air volume of 0.1 L/min, and speed of 400 rpm;
whole-process control: temperature of 33°C, pH of 7.0, tank pressure of 0 Mpa, air volume of 0.2 L/min, and speed of 400 rpm;
residual sugar control: 0.1-0.2% before F12h; ≤ 0.02% after F12h in combination with DO requirements;
culture maturity standard: OD₆₁₀ of 30-35; ventilation was stopped and static for 2 h after OD₆₁₀>30 (according to the purpose of batch experiments, thallus isolation or continuous catalysis was carried out);
material fed-batch: ammonia, 70% of concentrated sugar, and 5% of Paodi; and fermentation cycle: about 18-20 h.

The results showed that the L-valine yield of Corynebacterium glutamicum CGMCC No. 21260 was 84.1 g/L and OD₆₁₀ was 98.2; the L-valine yield of YPV-097 was 85.7 g/L, which was significantly higher than that of the strain CGMCC No. 21260 (P<0.05) and OD₆₁₀ was 99.2; and the L-valine yield of YPV-098 was 87.6 g/L, which was significantly higher than that of the strain CGMCC No. 21260 (P<0.05) and OD₆₁₀ was 100.3. It was indicated that replacing the original ilvC promoter with the yfjB or EP6 promoter in valine-producing bacteria CGMCC No. 21260 could increase the yield of valine, which was higher as the promotion of the expression of ilvC by the mutated EP6.

The present invention will be detailed above. For a person skilled in the art, the present invention may be implemented within a wide range under the same parameters, concentrations and conditions without departing from the purpose and scope of the present invention and without unnecessary experiments. Although special examples are given in the present invention, it should be understood that further improvements may be made to the present invention. In summary, according to the principles of the present invention, the present application is intended to include any change, use or improvement of the present invention, including changes that deviate from the scope disclosed in the present application and are made by conventional technologies known in the art. According to the scope of the claims attached below, some basic features can be applied.

Sequences 1-24 involved in the above examples were as follows:
**Sequence 1:** 
gaatttctccgcgtttttttcgcattcatctcgctaacttcgcttattatggggatcagtttcagggtttcaagg
gaagcactcacattgtcatcaatcttcgcaacaaggacctcggaaaa
**Sequence 2:** 
tctagagtcgacctgcaggcatgcaagcttggctgttttggcggatgagagaagattttcagcctgatacagatt
aaatcagaacgcagaagcggtctgataaaacagaatttgcctggcggcagtagcgcggtggtcccacctgacccc
atgccgaactcagaagtgaaacgccgtagcgccgatggtagtgtggggtctccccatgcgagagtagggaactgc
caggcatcaaataaaacgaaaggctcagtcgaaagactgggcctttcgttttatctgttgtttgtcggtgaacgc
tctcctgagtaggacaaatccgccgggagcggatttgaacgttgcgaagcaacggcccggagggtggcgggcagg
acgcccgccataaactgccaggcatcaaattaagcagaaggccatcctgacggatggcctttttgcgtttctaca
aactctttttgtttatttttctaaatacattcaaatatgtatccgctcatgaattaattccgctagatgacgtgc
ggcttcgacctcctgggcgtggcgcttgttggcgcgctcgcggctggctgcggcacgacacgcgtctgagcagta
ttttgcgcgccgtcctcgtgggtcaggccggggtgggatcaggccaccgcagtaggcgcagctgatgcgatcctc
cactactgcgcgtcctcctggcgctgccgagcacgcagctcgtcggccagctcttcaaggtcggccacaagcgtt
tctaggtcgctcgcggcacttgcccagtcgcgtgatgctggcgcgtctgtcgtatcgagggcgcggaaaaatccg
atcaccgtttttaaatcgacggcggcatcgagtgcgtcggactccagcgcgacatcggagagatccaccgctgat
gcttcaggccagttttggtacttcgtcgtgaaggtcatgacaccattataacgaacgttcgttaaaaattctagc
cccaattctgataatttcttccggcactcctgcgaaaacctgcgagacttcttgcccagaaaaaacgccaagcgc
agcggttaccgcactttttttccaggtgatttcaccctgaccagcgaagcggcactttagtgcatgaggtgtgcc
 
cctggtttcccctctttggagggttcaacccaaaaaagcacacaagcaaaaatgaaaatcatcatgagcaagttg
gtgcgaagcagcaacgcgctagctccaaaaaggtctccaggatctcgaggagatttttgagggggagggagtcga
ggaagagccagagcagaaggcggggaaccgttctctgccgacagcgtgagccccccttaaaaatcaggccgggga
ggaaccggggagggatcagagctaggagcgagacaccctaaagggggggaaccgttttctgctgacggtgtttcg
tttattagttttcagcccgtggatagcggagggtgagggcaagtgagagccagagcaaggacgggacccctaaag
gggggaaccgttttctgctgacggtgtttcgtttattagttttcagcccgtggacggccgcgtttagcttccatt
ccaagtgcctttctgacttgttggatgcgcctttcactgacacctagttcgcctgcaagctcacgagtcgaggga
tcagcaaccgattgagaacgggcatccaggatcgcagttttgacgcgaagttcgagcaactcgcctgtcatttct
cggcgtttgtttgcttccgctaatcgctgtcgcgtctcctgcgcatacttactttctgggtcagcccatctgcgt
gcattcgatgtagctgcgccccgtcgccccatcgtcgctagagctttccgccctcggctgctctgcgtttccacc
cgacgagcagggacgactggctggcctttagccacgtagccgcgcacacgacgcgccatcgtcaggcgatcacgc
atggcgggaagatccggctcccggccgtctgcaccgaccgcctgggcaacgttgtacgccacttcatacgcgtcg
atgatcttggcatcttttaggcgctcaccagcagctttgagctggtatcccacggtcaacgcgtggcgaaacgcg
gtctcgtcgcgcgctcgctctggatttgtccagagcactcgcacgccgtcgatcaggtcgccggacgcgtccagg
gcgctcggcaggctcgcgtccaaaatcgctagcgccttggcttctgcggtggcgcgttgtgccgcttcaatgcgg
gcgcgtccgctggaaaagtcctgctcaatgtactttttcggcttctgtgatccggtcatcgttcgagcaatctcc
attaggtcggccagccgatccacacgatcatgctggcagtgccatttataggctgtcggatcgtctgagacgtgc
agcggccaccggctcagcctatgcgaaaaagcctggtcagcgccgaaaacacgagtcatttcttccgtcgttgca
gccagcaggcgcatatttgggctggttttacctgctgcggcatacaccgggtcaatgagccagatgagctggcat
ttcccgctcagcggattcacgccgatccaagccggcgctttttctaggcgtgcccatttctctaaaatcgcgtag
acctgcgggtttacgtgctcaatcttcccgccggcctggtggctgggcacatcgatgtcaagcacgatcaccgcg
gcatgttgcgcgtgcgtcagcgcaacgtactggcaccgcgtcagcgcttttgagccagcccggtagagctttggt
tgggtttcgccggtatccgggtttttaatccaggcgctcgcgaaatctcttgtcttgctgccctggaagctttcg
cgtcccaggtgagcgagcagttcgcggcgatcttctgccgtccagccgcgtgagccgcagcgcatagcttcgggg
tgggtgtcgaacagatcggcggacaatttccacgcgctagctgtgactgtgtcctgcggatcggctagagtcatg
tcttgagtgctttctcccagctgatgactgggggttagccgacgccctgtgagttcccgctcacggggcgttcaa
ctttttcaggtatttgtgcagcttatcgtgttttcttcgtaaatgaacgcttaactaccttgttaaacgtggcaa
ataggcaggattgatggggatctagcttcacgctgccgcaagcactcagggcgcaagggctgctaaaggaagcgg
aacacgtagaaagccagtccgcagaaacggtgctgaccccggatgaatgtcagctactgggctatctggacaagg
gaaaacgcaagcgcaaagagaaagcaggtagcttgcagtgggcttacatggcgatagctagactgggcggtttta
tggacagcaagcgaaccggaattgccagctggggcgccctctggtaaggttgggaagccctgcaaagtaaactgg
atggctttcttgccgccaaggatctgatggcgcaggggatcaagatctgatcaagagacaggatgaggatcgttt
cgcatgattgaacaagatggattgcacgcaggttctccggccgcttgggtggagaggctattcggctatgactgg
gcacaacagacaatcggctgctctgatgccgccgtgttccggctgtcagcgcaggggcgcccggttctttttgtc
aagaccgacctgtccggtgccctgaatgaactccaagacgaggcagcgcggctatcgtggctggccacgacgggc
gttccttgcgcagctgtgctcgacgttgtcactgaagcgggaagggactggctgctattgggcgaagtgccgggg
caggatctcctgtcatctcaccttgctcctgccgagaaagtatccatcatggctgatgcaatgcggcggctgcat
acgcttgatccggctacctgcccattcgaccaccaagcgaaacatcgcatcgagcgagcacgtactcggatggaa
gccggtcttgtcgatcaggatgatctggacgaagagcatcaggggctcgcgccagccgaactgttcgccaggctc
aaggcgcggatgcccgacggcgaggatctcgtcgtgacccatggcgatgcctgcttgccgaatatcatggtggaa
aatggccgcttttctggattcatcgactgtggccggctgggtgtggcggaccgctatcaggacatagcgttggct
acccgtgatattgctgaagagcttggcggcgaatgggctgaccgcttcctcgtgctttacggtatcgccgctccc
gattcgcagcgcatcgccttctatcgccttcttgacgagttcttctgagcgggactctggggttcgcggaatcat
gaccaaaatcccttaacgtgagttttcgttccactgagcgtcagaccccgtagaaaagatcaaaggatcttcttg
agatcctttttttctgcgcgtaatctgctgcttgcaaacaaaaaaaccaccgctaccagcggtggtttgtttgcc
ggatcaagagctaccaactctttttccgaaggtaactggcttcagcagagcgcagataccaaatactgtccttct
agtgtagccgtagttaggccaccacttcaagaactctgtagcaccgcctacatacctcgctctgctaatcctgtt
 
accagtggctgctgccagtggcgataagtcgtgtcttaccgggttggactcaagacgatagttaccggataaggc
gcagcggtcgggctgaacggggggttcgtgcacacagcccagcttggagcgaacgacctacaccgaactgagata
cctacagcgtgagctatgagaaagcgccacgcttcccgaagggagaaaggcggacaggtatccggtaagcggcag
ggtcggaacaggagagcgcacgagggagcttccagggggaaacgcctggtatctttatagtcctgtcgggtttcg
ccacctctgacttgagcgtcgatttttgtgatgctcgtcaggggggcggagcctatggaaaaacgccagcaacgc
ggcctttttacggttcctggccttttgctggccttttgctcacatgttctttcctgcgttatcccctgattctgt
ggataaccgtattaccgcctttgagtgagctgataccgctcgccgcagccgaacgaccgagcgcagcgagtcagt
gagcgaggaagcggaagagcgcctgatgcggtattttctccttacgcatctgtgcggtatttcacaccgcatatg
gtgcactctcagtacaatctgctctgatgccgcatagttaagccagtatacactccgctatcgctacgtgactgg
gtcatggctgcgccccgacacccgccaacacccgctgacgcgccctgacgggcttgtctgctcccggcatccgct
tacagacaagctgtgaccgtctccgggagctgcatgtgtcagaggttttcaccgtcatcaccgaaacgcgcgagg
cagcagatcaattcgcgcgcgaaggcgaagcggcatgcatttacgttgacaccatcgaatggtgcaaaacctttc
gcggtatggcatgatagcgcccggaagagagtcaattcagggtggtgaatgtgaaaccagtaacgttatacgatg
tcgcagagtatgccggtgtctcttatcagaccgtttcccgcgtggtgaaccaggccagccacgtttctgcgaaaa
cgcgggaaaaagtggaagcggcgatggcggagctgaattacattcccaaccgcgtggcacaacaactggcgggca
aacagtcgttgctgattggcgttgccacctccagtctggccctgcacgcgccgtcgcaaattgtcgcggcgatta
aatctcgcgccgatcaactgggtgccagcgtggtggtgtcgatggtagaacgaagcggcgtcgaagcctgtaaag
cggcggtgcacaatcttctcgcgcaacgcgtcagtgggctgatcattaactatccgctggatgaccaggatgcca
ttgctgtggaagctgcctgcactaatgttccggcgttatttcttgatgtctctgaccagacacccatcaacagta
ttattttctcccatgaagacggtacgcgactgggcgtggagcatctggtcgcattgggtcaccagcaaatcgcgc
tgttagcgggcccattaagttctgtctcggcgcgtctgcgtctggctggctggcataaatatctcactcgcaatc
aaattcagccgatagcggaacgggaaggcgactggagtgccatgtccggttttcaacaaaccatgcaaatgctga
atgagggcatcgttcccactgcgatgctggttgccaacgatcagatggcgctgggcgcaatgcgcgccattaccg
agtccgggctgcgcgttggtgcggatatctcggtagtgggatacgacgataccgaagacagctcatgttatatcc
cgccgtcaaccaccatcaaacaggattttcgcctgctggggcaaaccagcgtggaccgcttgctgcaactctctc
agggccaggcggtgaagggcaatcagctgttgcccgtctcactggtgaaaagaaaaaccaccctggcgcccaata
cgcaaaccgcctctccccgcgcgttggccgattcattaatgcagctggcacgacaggtttcccgactggaaagcg
ggcagtgagcgcaacgcaattaatgtgagttagcgcgaattgatctggtttgacagcttatcatcgactgcacgg
tgcaccaatgcttctggcgtcaggcagccatcggaagctgtggtatggctgtgcaggtcgtaaatcactgcataa
ttcgtgtcgctcaaggcgcactcccgttctggataatgttttttgcgccgacatcataacggttctggcaaatat
tctgaaatgagctgttgacaattaatcatccggctcgtataatgtgtggaattgtgagcggataacaatttcaca
caggaaacagaccatggaattcgagctcggtacccggggatcctctaggaatttctccgcgtttttttcgcattc
atctcgctaacttcgcttattatggggatcagtttcagggtttcaagggaagcactcacattgtcatcaatcttc
gcaacaaggacctcggaaaaatgcgtaaaggagaagaagataacatggctatcattaaagagttcatgcgcttca
aagttcacatggagggttctgttaacggtcacgagttcgagatcgaaggcgaaggcgagggccgtccgtatgaag
gcacccagaccgccaaactgaaagtgactaaaggcggcccgctgccttttgcgtgggacatcctgagcccgcaat
ttatgtacggttctaaagcgtatgttaaacacccagcggatatcccggactatctgaagctgtcttttccggaag
gtttcaagtgggaacgcgtaatgaattttgaagatggtggtgtcgtgaccgtcactcaggactcctccctgcagg
atggcgagttcatctataaagttaaactgcgtggtactaattttccatctgatggcccggtgatgcagaaaaaga
cgatgggttgggaggcgtctagcgaacgcatgtatccggaagatggtgcgctgaaaggcgaaattaaacagcgcc
tgaaactgaaagatggcggccattatgacgctgaagtgaaaaccacgtacaaagccaagaaacctgtgcagctgc
ctggcgcgtacaatgtgaatattaaactggacatcacctctcataatgaagattatacgatcgtagagcaatatg
agcgcgcggagggtcgtcattctaccggtggcatggatgaactatacaaactagagtcgacctgcaggcatgcaa
gcttggctgttttggcgga
**Sequence 3:** 
Gaatttcttcgcgttttttttgcatccatcttgttaacttcgcttattatggggatcagtttccgggtttcaagg
gaagcggacacattgtcactatttcatttaacgcagggacctccaaatc
**Sequence 4:** 
tacggctccaaggacgtttgttttctgggtcagttaccccaaaaagcatatacagagaccaatgatttttcatta
aaaaggcagggatttgttataagtatgggtcgtattctgtgcgacgggtgtacctcggctagaatttctccccat
ggctacagttgaaaatttcaatgaacttcccgcacacgtatggccacgcaatgccgtgcgccaagaagacggcgt
tgtcaccgtcgctggtgtgcctctgcctgacctcgctgaagaatacggaaccccactgttcgtagtcgacgagga
cgatttccgttcccgctgtcgcgacatggctaccgcattcggtggaccaggcaatgtgcactacgcatctaaagc
gttcctgaccaagaccattgcacgttgggttgatgaagaggggctggcactggacattgcatccatcaacgaact
gggcattgccctggccgctggtttccccgccagccgtatcaccgcgcacggcaacaacaaaggcgtagagttcct
gcgcgcgttggttcaaaacggtgtgggacacgtggtgctggactccgcacaggaactagaactgttggattacgt
tgccgctggtgaaggcaagattcaggacgtgttgatccgcgtaaagccaggcatcgaagcacacacccacgagtt
catcgccactagccacgaagaccagaagttcggattctccctggcatccggttccgcattcgaagcagcaaaagc
cgccaacaacgcagaaaacctgaacctggttggcctgcactgccacgttggttcccaggtgttcgacgccgaagg
cttcaagctggcagcagaacgcgtgttgggcctgtactcacagatccacagcgaactgggcgttgcccttcctga
actggatctcggtggcggatacggcattgcctataccgcagctgaagaaccactcaacgtcgcagaagttgcctc
cgacctgctcaccgcagtcggaaaaatggcagcggaactaggcatcgacgcaccaaccgtgcttgttgagcccgg
ccgcgctatcgcaggcccctccaccgtgaccatctacgaagtcggcaccaccaaagacgtccacgtagacgacga
caaaacccgccgttacatcgccgtggacggaggcatgtccgacaacatccgcccagcactctacggctccgaata
cgacgcccgcgtagtatcccgcttcgccgaaggagacccagtaagcacccgcatcgtgggctcccactgcgaatc
cggcgatatcctgatcaacgatgaaatctacccatctgacatcaccagcggcgacttccttgcactcgcagccac
cggcgcatactgctacgccatgagctcccgctacaacgccttcacacggcccgccgtcgtgtccgtccgcgctgg
cagctcccgcctcatgctgcgccgcgaaacgctcgacgacatcctctcactagaggcataa
**Sequence 5:** 
cagtgccaagcttgcatgcctgcaggtcgactctagcccaggtgaagaagtcgttgagggagtttttgtagccct
gttcttgggaggccatgatgccgttccatgccaggctgggcgccattgcggaatcgaggacaaccttgtcggtgt
gctgtgggtagcgggtggcgtagaccgatccgaggtaggttccgtaggacagtccgaagatggagatcttgtcat
cgccaagtgcttggcggacgcgctcccagtcgttggcggtgttgtcggtggtcaggctggaggtgtagccggggg
tgccgatctcgcaggattctttaacgaaagcgccttcgcgggtgagcagcgagaggaaatcgtatcctggtgcga
tgttgtcgcagttaaccggtgtggagccgaccattccgcgaggctgcactgcaacgaggtcgtagttttggtaca
tggcttctggccagttcatggattggctgccgaagaagctataggcatcgccaccagggccaccggagttaccga
agatggtgccgtgcttttcgccttgggcagggaccttgacaaagcccacgctgatatcgccaagtgagggatcag
aatagtgcatgggcacgtcgatgctgccacattgagcggaggcaatatctacctgaggtgggcattcttcccagc
ggatgttttcttgcgctgctgcagtgggcattgataccaaaaaggggctaagcgcagtcgaggcggcaagaactg
ctactaccctttttattgtcgaacggggcatgaatttctccgcgtttttttcgcattcatctcgctaacttcgct
tattatggggatcagtttcagggtttcaagggaagcactcacattgtcatcaatcttcgcaacaaggacctcgga
aaaatgacaccagctgatctcgcaacattgattaaagagaccgcggtagaggttttgacctcccgcgagctcgat
acttctgttcttccggagcaggtagttgtggagcgtccgcgtaacccagagcacggcgattacgccaccaacatt
gcattgcaggtggctaaaaaggtcggtcagaaccctcgggatttggctacctggctggcagaggcattggctgca
gatgacgccattgattctgctgaaattgctggcccaggctttttgaacattcgccttgctgcagcagcacagggt
gaaattgtggccaagattctggcacagggcgagactttcggaaactccgatcacctttcccacttggacgtgaac
ctcgagttcgtttctgcaaacccaaccggacctattcaccttggcggaacccgctgggctgccgtgggtgactct
ttgggtcgtgtgctggaggcttccggcgcgaaagtgacccgcgaatactacttcaacgatcacggtcgccagatc
gatcgtttcgctttgtcccttcttgcagcggcgaagggcgagccaacgccagaagacggttatggcggcgaatac
attaaggaaattgcggaggcaatcgtcgaaaagcatcctgaagcgttggctttggagcctgccgcaacccaggag
cttttccgcgctgaaggcgtggagatgatgttcgagggtaccgagctcgaattcgtaatcatggtcatagctg
**Sequence 6:** 
cagtgccaagcttgcatgcctgcaggtcgactctagcccaggtgaagaagtcgttgagggagtttttgtagccct
gttcttgggaggccatgatgccgttccatgccaggctgggcgccattgcggaatcgaggacaaccttgtcggtgt
 
gctgtgggtagcgggtggcgtagaccgatccgaggtaggttccgtaggacagtccgaagatggagatcttgtcat
cgccaagtgcttggcggacgcgctcccagtcgttggcggtgttgtcggtggtcaggctggaggtgtagccggggg
tgccgatctcgcaggattctttaacgaaagcgccttcgcgggtgagcagcgagaggaaatcgtatcctggtgcga
tgttgtcgcagttaaccggtgtggagccgaccattccgcgaggctgcactgcaacgaggtcgtagttttggtaca
tggcttctggccagttcatggattggctgccgaagaagctataggcatcgccaccagggccaccggagttaccga
agatggtgccgtgcttttcgccttgggcagggaccttgacaaagcccacgctgatatcgccaagtgagggatcag
aatagtgcatgggcacgtcgatgctgccacattgagcggaggcaatatctacctgaggtgggcattcttcccagc
ggatgttttcttgcgctgctgcagtgggcattgataccaaaaaggggctaagcgcagtcgaggcggcaagaactg
ctactaccctttttattgtcgaacggggcatgaatttcttcgcgtttttttgcatccatcttgttaacttcgct
tattatggggatcagtttccgggtttcaagggaagcggacacattgtcactatttcatttaacgcaggacctcc
aaatcatgacaccagctgatctcgcaacattgattaaagagaccgcggtagaggttttgacctcccgcgagctcg
atacttctgttcttccggagcaggtagttgtggagcgtccgcgtaacccagagcacggcgattacgccaccaaca
ttgcattgcaggtggctaaaaaggtcggtcagaaccctcgggatttggctacctggctggcagaggcattggctg
cagatgacgccattgattctgctgaaattgctggcccaggctttttgaacattcgccttgctgcagcagcacagg
gtgaaattgtggccaagattctggcacagggcgagactttcggaaactccgatcacctttcccacttggacgtga
acctcgagttcgtttctgcaaacccaaccggacctattcaccttggcggaacccgctgggctgccgtgggtgact
ctttgggtcgtgtgctggaggcttccggcgcgaaagtgacccgcgaatactacttcaacgatcacggtcgccaga
tcgatcgtttcgctttgtcccttcttgcagcggcgaagggcgagccaacgccagaagacggttatggcggcgaat
acattaaggaaattgcggaggcaatcgtcgaaaagcatcctgaagcgttggctttggagcctgccgcaacccagg
agcttttccgcgctgaaggcgtggagatgatgttcgagggtaccgagctcgaattcgtaatcatggtcatagctg
**Sequence 7:** 
Ggcgacgatcttgtttgaccagttttggtacacggctagtggggtagtgccgaggccatacgtgtcgttgttttc
tgcaacccaggtgaagaagtcgttgagggagtttttgtagccctgttcttgggaggccatgatgccgttccatgc
caggctgggcgccattgcggaatcgaggacaaccttgtcggtgtgctgtgggtagcgggtggcgtagaccgatcc
gaggtaggttccgtaggacagtccgaagatggagatcttgtcatcgccaagtgcttggcggacgcgctcccagtc
gttggcggtgttgtcggtggtcaggctggaggtgtagccgggggtgccgatctcgcaggattctttaacgaaagc
gccttcgcgggtgagcagcgagaggaaatcgtatcctggtgcgatgttgtcgcagttaaccggtgtggagccgac
cattccgcgaggctgcactgcaacgaggtcgtagttttggtacatggcttctggccagttcatggattggctgcc
gaagaagctataggcatcgccaccagggccaccggagttaccgaagatggtgccgtgcttttcgccttgggcagg
gaccttgacaaagcccacgctgatatcgccaagtgagggatcagaatagtgcatgggcacgtcgatgctgccaca
ttgagcggaggcaatatctacctgaggtgggcattcttcccagcggatgttttcttgcgctgctgcagtgggcat
tgataccaaaaaggggctaagcgcagtcgaggcggcaagaactgctactaccctttttattgtcgaacggggcat
gaatttctccgcgtttttttcgcattcatctcgctaacttcgcttattatggggatcagt
**Sequence 8:** 
Ggcgacgatcttgtttgaccagttttggtacacggctagtggggtagtgccgaggccatacgtgtcgttgttttc
tgcaacccaggtgaagaagtcgttgagggagtttttgtagccctgttcttgggaggccatgatgccgttccatgc
caggctgggcgccattgcggaatcgaggacaaccttgtcggtgtgctgtgggtagcgggtggcgtagaccgatcc
gaggtaggttccgtaggacagtccgaagatggagatcttgtcatcgccaagtgcttggcggacgcgctcccagtc
gttggcggtgttgtcggtggtcaggctggaggtgtagccgggggtgccgatctcgcaggattctttaacgaaagc
gccttcgcgggtgagcagcgagaggaaatcgtatcctggtgcgatgttgtcgcagttaaccggtgtggagccgac
cattccgcgaggctgcactgcaacgaggtcgtagttttggtacatggcttctggccagttcatggattggctgcc
gaagaagctataggcatcgccaccagggccaccggagttaccgaagatggtgccgtgcttttcgccttgggcagg
gaccttgacaaagcccacgctgatatcgccaagtgagggatcagaatagtgcatgggcacgtcgatgctgccaca
ttgagcggaggcaatatctacctgaggtgggcattcttcccagcggatgttttcttgcgctgctgcagtgggcat
tgataccaaaaaggggctaagcgcagtcgaggcggcaagaactgctactaccctttttattgtcgaacggggcat
gaatttcttcgcgttttttttgcatccatcttgttaacttcgcttattatggggatcagt
**Sequence 9:** 
Cgcttattatggggatcagtttcagggtttcaagggaagcactcacattgtcatcaatcttcgcaacaaggacct
cggaaaaatgacaccagctgatctcgcaacattgattaaagagaccgcggtagaggttttgacctcccgcgagct
cgatacttctgttcttccggagcaggtagttgtggagcgtccgcgtaacccagagcacggcgattacgccaccaa
cattgcattgcaggtggctaaaaaggtcggtcagaaccctcgggatttggctacctggctggcagaggcattggc
tgcagatgacgccattgattctgctgaaattgctggcccaggctttttgaacattcgccttgctgcagcagcaca
gggtgaaattgtggccaagattctggcacagggcgagactttcggaaactccgatcacctttcccacttggacgt
gaacctcgagttcgtttctgcaaacccaaccggacctattcaccttggcggaacccgctgggctgccgtgggtga
ctctttgggtcgtgtgctggaggcttccggcgcgaaagtgacccgcgaatactacttcaacgatcacggtcgcca
gatcgatcgtttcgctttgtcccttcttgcagcggcgaagggcgagccaacgccagaagacggttatggcggcga
atacattaaggaaattgcggaggcaatcgtcgaaaagcatcctgaagcgttggctttggagcctgccgcaaccca
ggagcttttccgcgctgaaggcgtggagatgatgttcgagcacatcaaatcttccctgcatgagttcggcaccga
tttcgatgtctactac
**Sequence 10:** 
Cgcttattatggggatcagtttccgggtttcaagggaagcggacacattgtcactatttcatttaacgcagggac
ctccaaatcatgacaccagctgatctcgcaacattgattaaagagaccgcggtagaggttttgacctcccgcgag
ctcgatacttctgttcttccggagcaggtagttgtggagcgtccgcgtaacccagagcacggcgattacgccacc
aacattgcattgcaggtggctaaaaaggtcggtcagaaccctcgggatttggctacctggctggcagaggcattg
gctgcagatgacgccattgattctgctgaaattgctggcccaggctttttgaacattcgccttgctgcagcagca
cagggtgaaattgtggccaagattctggcacagggcgagactttcggaaactccgatcacctttcccacttggac
gtgaacctcgagttcgtttctgcaaacccaaccggacctattcaccttggcggaacccgctgggctgccgtgggt
gactctttgggtcgtgtgctggaggcttccggcgcgaaagtgacccgcgaatactacttcaacgatcacggtcgc
cagatcgatcgtttcgctttgtcccttcttgcagcggcgaagggcgagccaacgccagaagacggttatggcggc
gaatacattaaggaaattgcggaggcaatcgtcgaaaagcatcctgaagcgttggctttggagcctgccgcaacc
caggagcttttccgcgctgaaggcgtggagatgatgttcgagcacatcaaatcttccctgcatgagttcggcacc
gatttcgatgtctactac
**Sequence 11:** 
Ccccctctttaaggacagaaaggtttcaaccaagacatgttcgagctaatcgacgactggggtcccgaaaagatc
gtcatcgtcagcgatcaaaaaaccgggatgcgtggcgtacttgtcatcgacaacaccgcccgcggcatgggcaag
ggcggcacgcgcatgcagcccaccgtttcagtcgcagaaatagccaggttggctcgcgtcatgacctggaaatgg
gctggtgtagacctcttttatggtggtgcaaaagccggaatccaagcagaccccacctccccagataaagaagca
atccttcggtcattcgtcagaaaactctccaacgaagtacctaaagaatatgtcttcggcctggacatggggctg
actgaaaatgacgccgccatcatcgtcgacgagctcggctggggcaccagtatgggaacaccctacgagctcggt
ggagtgccctacgacaagcttggtatcaccggctttggtgttgcagaagtggtggatcaagtagcacaaatgcaa
aaactcaaaggtgcatcggtagcagtccaaggcttcggtgccgttggacatgccacagcttcccgcctggcagaa
cttggctatcctgttgtggctatctccacagcaaagggagcaatcgcagaccccaacgggctcaacatccccgag
ctcatggaactacgcgatcaggtgggtgactcacttgtggaccactacccagcacttcgcatcaacccaggtgac
gaacttttcaccgaagccgaaatcctcgtaccggcagcgctccaggacgtcatcgatgaagacgcagccaatcga
ctacaagcacaactcgtcgtcgaaggagcaaaccttcccactaatgaagccgcacaaaaagtcctaagtaaccgt
ggcatcactgtggttccagactttgtcgccaacgccggaggagtagtcgccgcagcattcgccatggataaccgc
atgtctgcattccgtgcagagactgccaacatcttcacgagcgtttccgacaaactccgatccaatgccgaaact
gtcctgaatttcacctctgaatctgacctgaccagccacgaagcggcaaggcaactttcacaggaacgagttctt
gcagctatgcgcgccagaggtatggttcgccgataa
**Sequence 12:** 
cagtgccaagcttgcatgcctgcaggtcgactctagcggaaacaacgcagccatagtcaccccagatgcagatct
tgacctggcacttcgcggaattgtcttcgcggctgccggaaccgctggtcagcgctgcaccaccatgcgccgcgt
tatcgtccacgagtccattgcagaagagctcaccgaaaaactcgtttccgcataccaaaccctgaccatcggcga
tcctcgcgacgagcagatcctcgttggaccactgatcaatgaatccggattccagggtatgcaggatgcactcaa
 
ggctgcaaccgaacagggcggaacggttatcaccggtggcaaccgagtcctcgaagatgaattcccagaggccta
ctacgttgagcctgcaatcgtaaccatgccagcacaaacagacattgtgcgcgatgaaaccttcgcaccaattct
ctacgtgctgacctactccactttggaagaagcaatcgcactccaaaacgatgttccacagggcctttcttctgc
aatcttcaccgaaaaccagcgcgaagcagagctcttcgtctccgcttccggctccgactgtggcattgccaacgt
caacatcggtacctccggcgcagaaatcggcggcgcattcggtggcgaaaaggaaaccggtggcggacgcgaatc
cggctccgactcctggaagtcctacatgcgacgcgccaccaacaccgtcaactactccggcgaactgccactcgc
ccaaggcgttaaattcctctaagaatttctccgcgtttttttcgcattcatctcgctaacttcgcttattatggg
gatcagtttcagggtttcaagggaagcactcacattgtcatcaatcttcgcaacaaggacctcggaaaaatgttc
gagctaatcgacgactggggtcccgaaaagatcgtcatcgtcagcgatcaaaaaaccgggatgcgtggcgtactt
gtcatcgacaacaccgcccgcggcatgggcaagggcggcacgcgcatgcagcccaccgtttcagtcgcagaaata
gccaggttggctcgcgtcatgacctggaaatgggctggtgtagacctcttttatggtggtgcaaaagccggaatc
caagcagaccccacctccccagataaagaagcaatccttcggtcattcgtcagaaaactctccaacgaagtacct
aaagaatatgtcttcggcctggacatggggctgactgaaaatgacgccgccatcatcgtcgacgagctcggctgg
ggcaccagtatgggaacaccctacgagctcggtggagtgccctacgacaagcttggtatcaccggctttggtgtt
gcagaagtggtggatcaagtagcacaaatgcaaaaactcaaaggtgcatcggtagcagtccaaggcttcggtgcc
gttggacatgccacagcttcccgcctggcagaacttggctatcctgttgtggctatctccacagcaaagggagca
atcgcagaccccaacgggctcaacatccccgagctcatggaactacgcgatcaggtgggtgactcacttgtggac
cactacccagcacttcgcatcaacccaggtgacgaacttttcaccgaagccgaaatcctcgtaccggcagcgctc
caggacgtcatgggtaccgagctcgaattcgtaatcatggtcatagctg
**Sequence 13:** 
cagtgccaagcttgcatgcctgcaggtcgactctagcggaaacaacgcagccatagtcaccccagatgcagatct
tgacctggcacttcgcggaattgtcttcgcggctgccggaaccgctggtcagcgctgcaccaccatgcgccgcgt
tatcgtccacgagtccattgcagaagagctcaccgaaaaactcgtttccgcataccaaaccctgaccatcggcga
tcctcgcgacgagcagatcctcgttggaccactgatcaatgaatccggattccagggtatgcaggatgcactcaa
ggctgcaaccgaacagggcggaacggttatcaccggtggcaaccgagtcctcgaagatgaattcccagaggccta
ctacgttgagcctgcaatcgtaaccatgccagcacaaacagacattgtgcgcgatgaaaccttcgcaccaattct
ctacgtgctgacctactccactttggaagaagcaatcgcactccaaaacgatgttccacagggcctttcttctgc
aatcttcaccgaaaaccagcgcgaagcagagctcttcgtctccgcttccggctccgactgtggcattgccaacgt
caacatcggtacctccggcgcagaaatcggcggcgcattcggtggcgaaaaggaaaccggtggcggacgcgaatc
cggctccgactcctggaagtcctacatgcgacgcgccaccaacaccgtcaactactccggcgaactgccactcgc
ccaaggcgttaaattcctctaagaatttcttcgcgttttttttgcatccatcttgttaacttcgcttattatggg
gatcagtttccgggtttcaagggaagcggacacattgtcactatttcatttaacgcagggacctccaaatcatgt
tcgagctaatcgacgactggggtcccgaaaagatcgtcatcgtcagcgatcaaaaaaccgggatgcgtggcgtac
ttgtcatcgacaacaccgcccgcggcatgggcaagggcggcacgcgcatgcagcccaccgtttcagtcgcagaaa
tagccaggttggctcgcgtcatgacctggaaatgggctggtgtagacctcttttatggtggtgcaaaagccggaa
tccaagcagaccccacctccccagataaagaagcaatccttcggtcattcgtcagaaaactctccaacgaagtac
ctaaagaatatgtcttcggcctggacatggggctgactgaaaatgacgccgccatcatcgtcgacgagctcggct
ggggcaccagtatgggaacaccctacgagctcggtggagtgccctacgacaagcttggtatcaccggctttggtg
ttgcagaagtggtggatcaagtagcacaaatgcaaaaactcaaaggtgcatcggtagcagtccaaggcttcggtg
ccgttggacatgccacagcttcccgcctggcagaacttggctatcctgttgtggctatctccacagcaaagggag
caatcgcagaccccaacgggctcaacatccccgagctcatggaactacgcgatcaggtgggtgactcacttgtgg
accactacccagcacttcgcatcaacccaggtgacgaacttttcaccgaagccgaaatcctcgtaccggcagcgc
tccaggacgtcatgggtaccgagctcgaattcgtaatcatggtcatagctg
**Sequence 14:** 
Gaagtaggtcctcgtgttgctgcccgattcggcaaaaccatcctggagctgggcggaaacaacgcagccatagtc
accccagatgcagatcttgacctggcacttcgcggaattgtcttcgcggctgccggaaccgctggtcagcgctgc
accaccatgcgccgcgttatcgtccacgagtccattgcagaagagctcaccgaaaaactcgtttccgcataccaa
 
acccctgaccatcggcgatcctcgcgacgagcagatcctcgttggaccactgatcaatgaatccggattccagggt
atgcaggatgcactcaaggctgcaaccgaacagggcggaacggttatcaccggtggcaaccgagtcctcgaagat
gaattcccagaggcctactacgttgagcctgcaatcgtaaccatgccagcacaaacagacattgtgcgcgatgaa
accttcgcaccaattctctacgtgctgacctactccactttggaagaagcaatcgcactccaaaacgatgttcca
cagggcctttcttctgcaatcttcaccgaaaaccagcgcgaagcagagctcttcgtctccgcttccggctccgac
tgtggcattgccaacgtcaacatcggtacctccggcgcagaaatcggcggcgcattcggtggcgaaaaggaaacc
ggtggcggacgcgaatccggctccgactcctggaagtcctacatgcgacgcgccaccaacaccgtcaactactcc
ggcgaactgccactcgcccaaggcgttaaattcctctaagaatttctccgcgtttttttcgcattcatctcgcta
acttcgcttattatggggatcagt
**Sequence 15:** 
Gaagtaggtcctcgtgttgctgcccgattcggcaaaaccatcctggagctgggcggaaacaacgcagccatagtc
accccagatgcagatcttgacctggcacttcgcggaattgtcttcgcggctgccggaaccgctggtcagcgctgc
accaccatgcgccgcgttatcgtccacgagtccattgcagaagagctcaccgaaaaactcgtttccgcataccaa
accctgaccatcggcgatcctcgcgacgagcagatcctcgttggaccactgatcaatgaatccggattccagggt
atgcaggatgcactcaaggctgcaaccgaacagggcggaacggttatcaccggtggcaaccgagtcctcgaagat
gaattcccagaggcctactacgttgagcctgcaatcgtaaccatgccagcacaaacagacattgtgcgcgatgaa
accttcgcaccaattctctacgtgctgacctactccactttggaagaagcaatcgcactccaaaacgatgttcca
cagggcctttcttctgcaatcttcaccgaaaaccagcgcgaagcagagctcttcgtctccgcttccggctccgac
tgtggcattgccaacgtcaacatcggtacctccggcgcagaaatcggcggcgcattcggtggcgaaaaggaaacc
ggtggcggacgcgaatccggctccgactcctggaagtcctacatgcgacgcgccaccaacaccgtcaactactcc
ggcgaactgccactcgcccaaggcgttaaattcctctaagaatttcttcgcgttttttttgcatccatcttgtta
acttcgcttattatggggatcagt
**Sequence 16:** 
Cgcttattatggggatcagtttcagggtttcaagggaagcactcacattgtcatcaatcttcgcaacaaggacct
cggaaaaatgttcgagctaatcgacgactggggtcccgaaaagatcgtcatcgtcagcgatcaaaaaaccgggat
gcgtggcgtacttgtcatcgacaacaccgcccgcggcatgggcaagggcggcacgcgcatgcagcccaccgtttc
agtcgcagaaatagccaggttggctcgcgtcatgacctggaaatgggctggtgtagacctcttttatggtggtgc
aaaagccggaatccaagcagaccccacctccccagataaagaagcaatccttcggtcattcgtcagaaaactctc
caacgaagtacctaaagaatatgtcttcggcctggacatggggctgactgaaaatgacgccgccatcatcgtcga
cgagctcggctggggcaccagtatgggaacaccctacgagctcggtggagtgccctacgacaagcttggtatcac
cggctttggtgttgcagaagtggtggatcaagtagcacaaatgcaaaaactcaaaggtgcatcggtagcagtcca
aggcttcggtgccgttggacatgccacagcttcccgcctggcagaacttggctatcctgttgtggctatctccac
agcaaagggagcaatcgcagaccccaacgggctcaacatccccgagctcatggaactacgcgatcaggtgggtga
ctcacttgtggaccactacccagcacttcgcatcaacccaggtgacgaacttttcaccgaagccgaaatcctcgt
accggcagcgctccaggacgtcatcgatgaagacgcagccaatcgactacaagcacaactcgtc
**Sequence 17:** 
Cgcttattatggggatcagtttccgggtttcaagggaagcggacacattgtcactatttcatttaacgcagggac
ctccaaatcatgttcgagctaatcgacgactggggtcccgaaaagatcgtcatcgtcagcgatcaaaaaaccggg
atgcgtggcgtacttgtcatcgacaacaccgcccgcggcatgggcaagggcggcacgcgcatgcagcccaccgtt
tcagtcgcagaaatagccaggttggctcgcgtcatgacctggaaatgggctggtgtagacctcttttatggtggt
gcaaaagccggaatccaagcagaccccacctccccagataaagaagcaatccttcggtcattcgtcagaaaactc
tccaacgaagtacctaaagaatatgtcttcggcctggacatggggctgactgaaaatgacgccgccatcatcgtc
gacgagctcggctggggcaccagtatgggaacaccctacgagctcggtggagtgccctacgacaagcttggtatc
accggctttggtgttgcagaagtggtggatcaagtagcacaaatgcaaaaactcaaaggtgcatcggtagcagtc
caaggcttcggtgccgttggacatgccacagcttcccgcctggcagaacttggctatcctgttgtggctatctcc
acagcaaagggagcaatcgcagaccccaacgggctcaacatccccgagctcatggaactacgcgatcaggtgggt
gactcacttgtggaccactacccagcacttcgcatcaacccaggtgacgaacttttcaccgaagccgaaatcctc
gtaccggcagcgctccaggacgtcatcgatgaagacgcagccaatcgactacaagcacaactcgtc
**Sequence 18:** 
acagcaattaatctgattgcacctgctgcataaatgtgactagtcaaacaccgtctaattgcatgtgtgtggtag
aacaataatgtagttgtctgcccaagcgagtttaactcccacgatttacagtgggggcagacatcttttcaccaa
aatttttacgaaaggcgagattttctcccatggctattgaactgctttatgatgctgacgctgacctctccttga
tccagggccgcaaggttgccatcgttggctacggctcccagggccacgcacactcccagaacctccgcgattctg
gcgttgaggttgtcattggtctgcgcgagggctccaagtccgcagagaaggcaaaggaagcaggcttcgaggtca
agaccaccgctgaggctgcagcttgggctgacgtcatcatgctcctggctccagacacctcccaggcagaaatct
tcaccaacgacatcgagccaaacctgaacgcaggcgacgcactgctgttcggccacggcctgaacattcacttcg
acctgatcaagccagctgacgacatcatcgttggcatggttgcgccaaagggcccaggccacttggttcgccgtc
agttcgttgatggcaagggtgttccttgcctcatcgcagtcgaccaggacccaaccggaaccgcacaggctctga
ccctgtcctacgcagcagcaatcggtggcgcacgcgcaggcgttatcccaaccaccttcgaagctgagaccgtca
ccgacctcttcggcgagcaggctgttctctgcggtggcaccgaggaactggtcaaggttggcttcgaggttctca
ccgaagctggctacgagccagagatggcatacttcgaggttcttcacgagctcaagctcatcgttgacctcatgt
tcgaaggtggcatcagcaacatgaactactctgtttctgacaccgctgagttcggtggctacctctccggcccac
gcgtcatcgatgcagacaccaagtcccgcatgaaggacatcctgaccgatatccaggacggcaccttcaccaagc
gcctcatcgcaaacgttgagaacggcaacaccgaacttgagggccttcgtgcttcctacaacaaccacccaatcg
aggagaccggcgctaagctccgcgacctcatgagctgggtcaaggttgacgctcgcgcagaaaccgcttaa
**Sequence 19:** 
cagtgccaagcttgcatgcctgcaggtcgactctagagagatcaacgaccgcccagtagtcatcgacttcatcgt
cggtgaagacgcacaggtatggccaatggtgtctgctggatcatccaactccgatatccagtacgcactcggatt
gcgcccattctttgatggtgatgaatctgcagcagaagatcctgccgacattcacgaagccgtcagcgacattga
tgccgccgttgaatcgaccgaggcataaggagagacccaagatggctaattctgacgtcacccgccacatcctgt
ccgtactcgttcaggacgtagacggaatcatttcccgcgtatcaggtatgttcacccgacgcgcattcaacctcg
tgtccctcgtgtctgcaaagaccgaaacactcggcatcaaccgcatcacggttgttgtcgacgccgacgagctca
acattgagcagatcaccaagcagctcaacaagctgatccccgtgctcaaagtcgtgcgacttgatgaagagacca
ccatcgcccgcgcaatcatgctggttaaggtctctgcggatagcaccaaccgtccgcagatcgtcgacgccgcga
acatcttccgcgcccgagtcgtcgacgtggctccagactctgtggttattgaatccacaggcaccccaggcaagc
tccgcgcactgcttgatgtgatggaaccattcggaatccgcgaactgatccaatccggacagattgcactcaacc
gcggtccgaagaccatggctccggccaagatctaagaatttctccgcgtttttttcgcattcatctcgctaactt
cgcttattatggggatcagtttcagggtttcaagggaagcactcacattgtcatcaatcttcgcaacaaggacct
cggaaaaatggctattgaactgctttatgatgctgacgctgacctctccttgatccagggccgcaaggttgccat
cgttggctacggctcccagggccacgcacactcccagaacctccgcgattctggcgttgaggttgtcattggtct
gcgcgagggctccaagtccgcagagaaggcaaaggaagcaggcttcgaggtcaagaccaccgctgaggctgcagc
ttgggctgacgtcatcatgctcctggctccagacacctcccaggcagaaatcttcaccaacgacatcgagccaaa
cctgaacgcaggcgacgcactgctgttcggccacggcctgaacattcacttcgacctgatcaagccagctgacga
catcatcgttggcatggttgcgccaaagggcccaggccacttggttcgccgtcagttcgttgatggcaagggtgt
tccttgcctcatcgcagtcgaccaggacccaaccggaaccgcacaggctctgaccctgtcctacgcagcagcaat
cggtggcgcacgcgcaggcgttatcccaaccaccttcgaagctgagaccgtcaccgacctcttcggcgagcaggc
tgttctctgcggtggcaccgaggaactggtcaaggttggcttcgaggttctcaccgaagctggctacgagccaga
gatggcatacttcgaggttcttcacgagctcaagctcatcgttgacctcatgttcgaaggtggcatcagcaacat
gaactactctgtttctgacaccgctgagttcggtggctacctctccggcccacgcgtcatcgatgcagacaccaa
gtcccgcatgaaggacatcctgaccgatatccaggacggcaccttcaccaagcgcctcagggtaccgagctcgaa
ttcgtaatcatggtcatagctg
**Sequence 20:** 
cagtgccaagcttgcatgcctgcaggtcgactctagagagatcaacgaccgcccagtagtcatcgacttcatcgt
cggtgaagacgcacaggtatggccaatggtgtctgctggatcatccaactccgatatccagtacgcactcggatt
 
gcgcccattctttgatggtgatgaatctgcagcagaagatcctgccgacattcacgaagccgtcagcgacattga
tgccgccgttgaatcgaccgaggcataaggagagacccaagatggctaattctgacgtcacccgccacatcctgt
ccgtactcgttcaggacgtagacggaatcatttcccgcgtatcaggtatgttcacccgacgcgcattcaacctcg
tgtccctcgtgtctgcaaagaccgaaacactcggcatcaaccgcatcacggttgttgtcgacgccgacgagctca
acattgagcagatcaccaagcagctcaacaagctgatccccgtgctcaaagtcgtgcgacttgatgaagagacca
ccatcgcccgcgcaatcatgctggttaaggtctctgcggatagcaccaaccgtccgcagatcgtcgacgccgcga
acatcttccgcgcccgagtcgtcgacgtggctccagactctgtggttattgaatccacaggcaccccaggcaagc
tccgcgcactgcttgatgtgatggaaccattcggaatccgcgaactgatccaatccggacagattgcactcaacc
gcggtccgaagaccatggctccggccaagatctaagaatttcttcgcgttttttttgcatccatcttgttaactt
cgcttattatggggatcagtttccgggtttcaagggaagcggacacattgtcactatttcatttaacgcagggac
ctccaaatcatggctattgaactgctttatgatgctgacgctgacctctccttgatccagggccgcaaggttgcc
atcgttggctacggctcccagggccacgcacactcccagaacctccgcgattctggcgttgaggttgtcattggt
ctgcgcgagggctccaagtccgcagagaaggcaaaggaagcaggcttcgaggtcaagaccaccgctgaggctgca
gcttgggctgacgtcatcatgctcctggctccagacacctcccaggcagaaatcttcaccaacgacatcgagcca
aacctgaacgcaggcgacgcactgctgttcggccacggcctgaacattcacttcgacctgatcaagccagctgac
gacatcatcgttggcatggttgcgccaaagggcccaggccacttggttcgccgtcagttcgttgatggcaagggt
gttccttgcctcatcgcagtcgaccaggacccaaccggaaccgcacaggctctgaccctgtcctacgcagcagca
atcggtggcgcacgcgcaggcgttatcccaaccaccttcgaagctgagaccgtcaccgacctcttcggcgagcag
gctgttctctgcggtggcaccgaggaactggtcaaggttggcttcgaggttctcaccgaagctggctacgagcca
gagatggcatacttcgaggttcttcacgagctcaagctcatcgttgacctcatgttcgaaggtggcatcagcaac
atgaactactctgtttctgacaccgctgagttcggtggctacctctccggcccacgcgtcatcgatgcagacacc
aagtcccgcatgaaggacatcctgaccgatatccaggacggcaccttcaccaagcgcctcagggtaccgagctcg
aattcgtaatcatggtcatagctg
**Sequence 21:** 
Cccgactttgttaccctttctgagggacttggctgtgttgccatccgcgtcaccaaagcggaggaagtactgcca
gccatccaaaaggctcgagagatcaacgaccgcccagtagtcatcgacttcatcgtcggtgaagacgcacaggta
tggccaatggtgtctgctggatcatccaactccgatatccagtacgcactcggattgcgcccattctttgatggt
gatgaatctgcagcagaagatcctgccgacattcacgaagccgtcagcgacattgatgccgccgttgaatcgacc
gaggcataaggagagacccaagatggctaattctgacgtcacccgccacatcctgtccgtactcgttcaggacgt
agacggaatcatttcccgcgtatcaggtatgttcacccgacgcgcattcaacctcgtgtccctcgtgtctgcaaa
gaccgaaacactcggcatcaaccgcatcacggttgttgtcgacgccgacgagctcaacattgagcagatcaccaa
gcagctcaacaagctgatccccgtgctcaaagtcgtgcgacttgatgaagagaccaccatcgcccgcgcaatcat
gctggttaaggtctctgcggatagcaccaaccgtccgcagatcgtcgacgccgcgaacatcttccgcgcccgagt
cgtcgacgtggctccagactctgtggttattgaatccacaggcaccccaggcaagctccgcgcactgcttgatgt
gatggaaccattcggaatccgcgaactgatccaatccggacagattgcactcaaccgcggtccgaagaccatggc
tccggccaagatctaagaatttctccgcgtttttttcgcattcatctcgctaacttcgcttattatggggatcag
t
**Sequence 22:** 
Cccgactttgttaccctttctgagggacttggctgtgttgccatccgcgtcaccaaagcggaggaagtactgcca
gccatccaaaaggctcgagagatcaacgaccgcccagtagtcatcgacttcatcgtcggtgaagacgcacaggta
tggccaatggtgtctgctggatcatccaactccgatatccagtacgcactcggattgcgcccattctttgatggt
gatgaatctgcagcagaagatcctgccgacattcacgaagccgtcagcgacattgatgccgccgttgaatcgacc
gaggcataaggagagacccaagatggctaattctgacgtcacccgccacatcctgtccgtactcgttcaggacgt
agacggaatcatttcccgcgtatcaggtatgttcacccgacgcgcattcaacctcgtgtccctcgtgtctgcaaa
gaccgaaacactcggcatcaaccgcatcacggttgttgtcgacgccgacgagctcaacattgagcagatcaccaa
gcagctcaacaagctgatccccgtgctcaaagtcgtgcgacttgatgaagagaccaccatcgcccgcgcaatcat
gctggttaaggtctctgcggatagcaccaaccgtccgcagatcgtcgacgccgcgaacatcttccgcgcccgagt
 
cgtcgacgtggctccagactctgtggttattgaatccacaggcaccccaggcaagctccgcgcactgcttgatgt
gatggaaccattcggaatccgcgaactgatccaatccggacagattgcactcaaccgcggtccgaagaccatggc
tccggccaagatctaagaatttcttcgcgttttttttgcatccatcttgttaacttcgcttattatggggatcag
t
**Sequence 23:** 
Cgcttattatggggatcagtttcagggtttcaagggaagcactcacattgtcatcaatcttcgcaacaaggacct
cggaaaaatggctattgaactgctttatgatgctgacgctgacctctccttgatccagggccgcaaggttgccat
cgttggctacggctcccagggccacgcacactcccagaacctccgcgattctggcgttgaggttgtcattggtct
gcgcgagggctccaagtccgcagagaaggcaaaggaagcaggcttcgaggtcaagaccaccgctgaggctgcagc
ttgggctgacgtcatcatgctcctggctccagacacctcccaggcagaaatcttcaccaacgacatcgagccaaa
cctgaacgcaggcgacgcactgctgttcggccacggcctgaacattcacttcgacctgatcaagccagctgacga
catcatcgttggcatggttgcgccaaagggcccaggccacttggttcgccgtcagttcgttgatggcaagggtgt
tccttgcctcatcgcagtcgaccaggacccaaccggaaccgcacaggctctgaccctgtcctacgcagcagcaat
cggtggcgcacgcgcaggcgttatcccaaccaccttcgaagctgagaccgtcaccgacctcttcggcgagcaggc
tgttctctgcggtggcaccgaggaactggtcaaggttggcttcgaggttctcaccgaagctggctacgagccaga
gatggcatacttcgaggttcttcacgagctcaagctcatcgttgacctcatgttcgaaggtggcatcagcaacat
gaactactctgtttctgacaccgctgagttcggtggctacctctccggcccacgcgtcatcgatgcagacaccaa
gtcccgcatgaaggacatcctgaccgatatccaggacggcaccttcaccaagcgcctcatcgcaaacgttgagaa
cggcaacaccgaacttgagggccttcgtgcttcctacaacaaccaccc
**Sequence 24:**

cgcttattatggggatcagtttccgggtttcaagggaagcggacacattgtcactatttcatttaacgcagggac
ctccaaatcatggctattgaactgctttatgatgctgacgctgacctctccttgatccagggccgcaaggttgcc
atcgttggctacggctcccagggccacgcacactcccagaacctccgcgattctggcgttgaggttgtcattggt
ctgcgcgagggctccaagtccgcagagaaggcaaaggaagcaggcttcgaggtcaagaccaccgctgaggctgca
gcttgggctgacgtcatcatgctcctggctccagacacctcccaggcagaaatcttcaccaacgacatcgagcca
aacctgaacgcaggcgacgcactgctgttcggccacggcctgaacattcacttcgacctgatcaagccagctgac
gacatcatcgttggcatggttgcgccaaagggcccaggccacttggttcgccgtcagttcgttgatggcaagggt
gttccttgcctcatcgcagtcgaccaggacccaaccggaaccgcacaggctctgaccctgtcctacgcagcagca
atcggtggcgcacgcgcaggcgttatcccaaccaccttcgaagctgagaccgtcaccgacctcttcggcgagcag
gctgttctctgcggtggcaccgaggaactggtcaaggttggcttcgaggttctcaccgaagctggctacgagcca
gagatggcatacttcgaggttcttcacgagctcaagctcatcgttgacctcatgttcgaaggtggcatcagcaac
atgaactactctgtttctgacaccgctgagttcggtggctacctctccggcccacgcgtcatcgatgcagacacc
aagtcccgcatgaaggacatcctgaccgatatccaggacggcaccttcaccaagcgcctcatcgcaaacgttgag
aacggcaacaccgaacttgagggccttcgtgcttcctacaacaaccaccc

### Industrial applications

For amino acid-producing bacteria, in the present invention, the EP6 promoter promotes the genes in the corresponding amino acid synthesis pathways to obtain the corresponding expression cassette. The expression cassette is used as an exogenous strong promoter to be integrated into bacteria with high-yield amino acids to replace an original promoter of key genes for amino acid synthesis, so that the yield of a corresponding amino acid is improved. It is noted that the EP6 promoter of the present invention not only has high starting activity, but also can be used for the production of amino acids, and has a very important application prospect.

## Claims

1. A DNA molecule, comprising a sequence set forth in SEQ ID No. 3 in the sequence listing.

2. A biomaterial associated with the DNA molecule according to claim 1, which is any one of the followings B1) to B7):
B1) an expression cassette comprising the DNA molecule according to claim 1;
B2) a recombinant vector comprising the DNA molecule according to claim 1;
B3) a recombinant vector comprising the expression cassette of B1);
B4) a recombinant microorganism comprising the DNA molecule according to claim 1;
B5) a recombinant microorganism comprising the expression cassette of B1);
B6) a recombinant microorganism comprising the recombinant vector of B2); and
B7) a recombinant microorganism comprising the recombinant vector of B3).

3. Use of the DNA molecule according to claim 1 as a promoter.

4. Use of the DNA molecule according to claim 1 or the biomaterial according to claim 2 in the production of an amino acid.

5. The use according to claim 4, wherein the amino acid is lysine, glutamic acid or valine.

6. A method for producing an amino acid, comprising: introducing the DNA molecule according to claim 1 into a biological cell capable of synthesizing a target amino acid, so that the DNA molecule according to claim 1 drives the expression of a gene in a synthesis pathway of the target amino acid in the biological cell to obtain a recombinant biological cell; and culturing the recombinant biological cell to obtain the target amino acid.

7. The method according to claim 6, wherein the biological cell is yeast, bacteria, algae, fungus, a plant cell or an animal cell that can synthesize the target amino acid.

8. The method according to claim 7, wherein the bacteria are *Corynebacterium glutamicum.*

9. The method according to any one of claims 6 to 8, wherein the target amino acid is lysine, glutamic acid or valine.

10. A product for producing an amino acid, an active ingredient thereof is the DNA molecule according to claim 1 or the biomaterial according to claim 2.
